(19) Europäisches Patentamt
European Patent Office
Office européen des brevets

(11) Veröffentlichungsnummer : **0 391 185 B1**

(12) # EUROPÄISCHE PATENTSCHRIFT

(45) Veröffentlichungstag der Patentschrift : **29.12.93 Patentblatt 93/52**

(51) Int. Cl.$^5$ : **C07D 471/04,** A61K 31/435, // (C07D471/04, 221:00, 221:00)

(21) Anmeldenummer : **90105652.3**

(22) Anmeldetag : **24.03.90**

(54) **Substituierte 1,8-Naphthyridine.**

Die Akte enthält technische Angaben, die nach dem Eingang der Anmeldung eingereicht wurden und die nicht in dieser Patentschrift enthalten sind.

(30) Priorität : **06.04.89 DE 3911064**

(43) Veröffentlichungstag der Anmeldung : **10.10.90 Patentblatt 90/41**

(45) Bekanntmachung des Hinweises auf die Patenterteilung : **29.12.93 Patentblatt 93/52**

(84) Benannte Vertragsstaaten : **AT BE CH DE DK ES FR GB GR IT LI LU NL SE**

(56) Entgegenhaltungen :
**EP-A- 0 022 478**
**EP-A- 0 114 027**
**DE-A- 2 322 350**
**DE-A- 3 825 611**
**US-A- 4 613 610**

(73) Patentinhaber : **BAYER AG**
**D-51368 Leverkusen (DE)**

(72) Erfinder : **Hübsch, Walter, Dr.**
**Am Eckbusch 43/50**
**D-5600 Wuppertal 1 (DE)**
Erfinder : **Angerbauer, Rolf, Dr.**
**Moospfad 20**
**D-5600 Wuppertal 1 (DE)**
Erfinder : **Fey, Peter, Dr.**
**Ursulastrasse 14**
**D-5600 Wuppertal 1 (DE)**
Erfinder : **Philipps, Thomas, Dr.**
**Silesiusstrasse 74**
**D-5000 Köln 80 (DE)**
Erfinder : **Bischoff, Hilmar, Dr.**
**Am Rohm 78**
**D-5600 Wuppertal 1 (DE)**
Erfinder : **Petzinna, Dieter, Dr., Dr.**
**Peter-Berten-Strasse 10**
**D-4000 Düsseldorf 12 (DE)**
Erfinder : **Schmidt, Delf, Dr.**
**Am Eckbusch 55b**
**D-5600 Wuppertal 1 (DE)**

EP 0 391 185 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue substituierte 1,8-Naphthyridine, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl-Coenzym A Reduktase (HMG-CoA- Reduktase) sind [Mevinolin, EP-A 22 478; US 4 231 938]. Darüberhinaus sind auch bestimmte Indolderivate bzw. Pyrazolderivate Inhibitoren der HMG-CoA-Reduktase [EP-A 1 114 027; US-Patent 4 613 610].

Es wurden nun neue substituierte 1,8-Naphthyridine der allgemeinen Formeln (Ia,b)

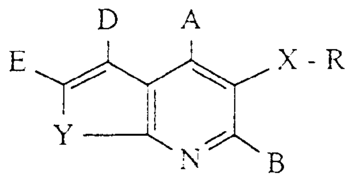

(Ia)

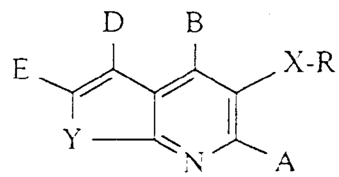

(Ib)

in weicher

A - für Pyridyl oder Pyrimidyl steht, das gegebenenfalls bis zu 2-fach gleich oder verschieden durch Fluor, Chlor, Brom, Trifluormethyl, geradkettiges oder verzweigtes Alkyl mit bis zu 6 Kohlenstoffatomen oder Phenyl substituiert ist,
- für Phenyl oder Naphthyl steht, das gegebenenfalls bis zu 4-fach gleich oder verschieden substituiert ist durch geradkettiges oder verzweigtes Alkyl, Alkylthio, Alkylsulfonyl, Alkoxy oder Alkoxycarbonyl mit jeweils bis zu 8 Kohlenstoffatomen, welche ihrerseits durch Hydroxy. Alkoxy mit bis zu 4 kohlenstoffatomen, Phenyl oder durch eine Gruppe der Formel $-NR^1R^2$ substituiert sein können,
worin
$R^1$ und $R^2$ gleich oder verschieden sind und
- Wasserstoff, Phenyl, Phenylsulfonyl, geradkettiges oder verzweigtes Alkyl oder Alkylsulfonyl mit bis zu 6 Kohlenstoffatomen, Benzyl oder Benzylsulfonyl bedeuten, oder
- eine Gruppe der Formel $-COR^3$ bedeuten,
worin

$R^3$ - geradkettiges oder verzweigtes Alkyl oder Alkoxy mit bis zu 6 Kohlenstoffatomen oder Phenyl bedeutet, oder durch Phenyl, Phenyloxy, Fluor, Chlor, Brom, Nitro, Cyano, Trifluormethyl, Trifluormethoxy,Benzyloxy oder durch eine Gruppe der Formel $-NR^1R^2$ substituiert ist,
worin
$R^1$ und $R^2$ die oben angegebene Bedeutung haben,

B - für Cyclopropyl, Cyclobutyl, Cyclopentyl oder Cyclohexyl steht,
- für geradkettiges oder verzweigtes Alkyl mit bis zu 10 Kohlenstoffatomen steht, das gebenenfalls durch Fluor, Chlor, Brom, Trifluormethyl oder Methylthio substituiert sein kann,
D und E gleich oder verschieden sind und
- für Wasserstoff, Hydroxyl, Alkoxy mit bis zu 6 Kohlenstoffatomen, geradkettiges oder verzweigtes Alkyl mit bis zu 8 Kohlenstoffatomen, Phenyl oder für eine Gruppe der Formel
$-NR^1R^2$ stehen,
worin
$R^1$ und $R^2$ die oben angegebene Bedeutung haben,

Y - für eine Gruppe der Formel

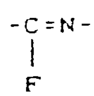

oder

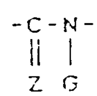

steht
worin

F - Wasserstoff, Hydroxy, Mercapto, Fluor, Chlor oder Brom bedeutet, oder geradkettiges oder verzweigtes Alkyl, Akloxy oder Alkylthio mit bis zu 8 Kohlenstoffatomen bedeutet, die gegebenenfalls durch Phenyl substituiert sind oder Phenoxy, Benzyloxy oder eine Gruppe der Formel $-NR^1R^2$ bedeutet,
worin
$R^1$ und $R^2$ die oben angegebene Bedeutung haben,

Z - Sauerstoff oder Schwefel bedeutet,

G - Wasserstoff, geradkettiges oder verzweigtes Alkyl oder Alkenyl mit jeweils bis zu 8 Kohlenstoffatomen bedeutet, das gegebenenfalls durch Fluor, Chlor, Brom, Cyano, Alkoxy mit bis zu 6 Kohlenstoffatomen, Phenyl, Phenoxy, Benyzloxy, Pyrryl, Furyl oder durch eine Gruppe der Formel $-NR^1R^2$, $-COR^3$ oder $-COOR^4$ substituiert ist,
worin
$R^1$, $R^2$ und $R^3$ die oben angegebene Bedeutung haben,

$R^4$ - Wasserstoff, geradkettiges oder verzweigtes Alkyl mit bis zu 8 Kohlenstoffatomen bedeutet, das gegebenenfalls durch Hydroxy, Phenyl, Fluor, Chlor oder Brom substituiert ist,
- Phenyl bedeutet, welches seinerseits durch Fluor, Chlor, Brom oder Hydroxy substituiert sein kann,

X - für eine Gruppe der Formel $-CH_2-CH_2-$ oder $-CH=CH-$steht
und

R - für eine Gruppe der Formel

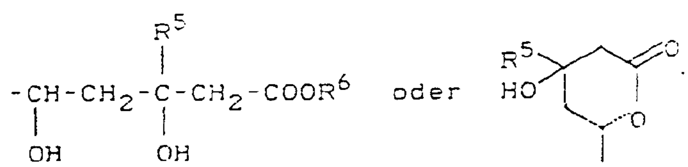

steht,
worin

$R^5$ - Wasserstoff oder geradkettiges oder verzweigtes Alkyl mit bis zu 8 Kohlenstoffatomen bedeutet und

$R^6$ - Wasserstoff oder geradkettiges oder verzweigtes Alkyl mit bis zu 8 Kohlenstoffatomen oder Benzyl bedeutet, oder
- Phenyl oder ein Kation bedeutet
und deren Salze gefunden.

Bildet $R^6$ mit der Carboxygruppe einen Esterrest so ist hiermit bevorzugt ein physiologisch verträglicher Esterrest gemeint

Hierzu gehören beispielsweise Alkylester ($C_1$ bis $C_6$) und Aralkylester ($C_7$ bis $C_{10}$), bevorzugt ($C_1$-$C_4$)-Alkylester sowie Benzylester. Darüberhinaus seien die folgenden Esterreste genannt : Methylester, Ethylester, Propylester, Benzylester.

Steht $R^6$ für ein Kation, so ist bevorzugt ein physiologisch verträgliches Metall- oder Ammoniumkation gemeint, Bevorzugt sind hierbei Alkali- bzw. Erdalkalikationen wie beispielsweise Natrium-, Kalium-, Magnesium- oder Calciumkationen, sowie Aluminium- oder Ammoniumkationen, sowie nichttoxische substituierte Ammoniumkationen aus Aminen wie ($C_1$-$C_4$)-Dialkylamine, ($C_1$-$C_4$)-Trialkylamine, Prokain, Dibenzylamin, N,N'-Dibenzylethylendiamin, N-Benzyl-β-phenylethylamin, N-Methylmorpholin oder N-Ethylmorpholin, 1-Ephenamin, Dihydroabietylamin, N,N'-Bis-dihydroabietylethylendiamin, N-Niederalkylpiperidin und andere Amine, die zur Bildung von Salzen verwendet werden können.

Überraschenderweise zeigen die erfindungsgemäßen substituierten 1,8-Naphthyridine eine überlegene inhibitorische Wirkung auf die HMG-CoA-Reduktase (3-Hydroxy-3-methyl-glutaryl-Coenzym A Reduktase).

Bevorzugt sind Verbindungen der allgemeinen Formeln (Ia) und (Ib),
in welchen

A - für Phenyl steht, das gegebenenfalls bis zu 3-fach gleich oder verschieden substituiert ist durch geradkettiges oder verzweigtes Alkyl mit bis zu 6 Kohlenstoffatomen, welches seinerseits durch Hydroxy, Methoxy, Ethoxy, Propoxy oder Phenyl substituiert sein kann,
oder durch Phenyl, Phenoxy, Fluor, Chlor, Brom oder Benzyloxy substituiert ist,

B - für Cyclopropyl, Cyclobutyl, Cyclopentyl oder Cyclohexyl steht,
- für Methyl, Ethyl, Propyl, Isopropyl, Butyl, Isobutyl, tert.Butyl oder Trifluormethyl steht,

D und E gleich oder verschieden sind und
- für Wasserstoff, Hydroxy, Methyl, Ethyl, Propyl, Isopropyl, Methoxy oder Ethoxy stehen,

Y - für eine Gruppe der Formel

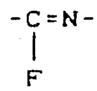 oder 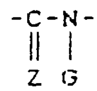

steht,
worin

F - Wasserstoff, Hydroxy, Fluor oder Chlor bedeutet, oder geradkettiges oder verzweigtes Alkyl, Alkoxy oder Alkylthio mit bis zu 6 Kohlenstoffatomen bedeutet, die gegebenenfalls durch Phenyl substituiert sind, oder Benzyloxy oder eine Gruppe der Formel $-NR^1R^2$ bedeutet, worin
$R^1$ und $R^2$ gleich oder verschieden sind und
- Wasserstoff oder geradkettiges oder verzweigtes Alkyl mit bis zu 4 Kohlenstoffatomen oder Benzyl bedeuten,

Z - Sauerstoff oder Schwefel bedeutet,

G - Wasserstoff, geradkettiges oder verzweigtes Alkyl mit bis zu Kohlenstoffatomen bedeutet, das gegebenenfalls durch Fluor, Chlor, Cyano, Alkoxy mit bis zu 4 Kohlenstoffatomen, Phenyl, Benzyloxy oder durch eine Gruppe der Formel $-COR^3$ oder $-COOR^4$ substituiert ist,
worin
$R^3$ - geradkettiges oder verzweigtes Alkyl oder Phenyl bedeutet,
$R^4$ - Wasserstoff, geradkettiges oder verzweigtes Alkyl mit bis zu 6 Kohlenstoffatomen oder Phenyl bedeutet,

X - für eine Gruppe -CH=CH- oder $-CH_2-CH_2-$ steht
und

R - für eine Gruppe der Formel

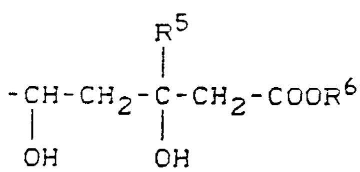 oder 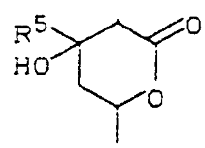

steht,
worin

$R^5$ - Wasserstoff, Methyl, Ethyl, Propyl, Isopropyl, Butyl, Isobutyl oder tert.Butyl bedeutet
und

$R^6$ - Wasserstoff, Methyl, Ethyl, Propyl, Isopropyl, Butyl, Isobutyl, tert.Butyl oder Benzyl bedeutet, oder
- ein Natrium-, Kalium-, Calcium-, Magnesium- oder Ammoniumion bedeutet
und deren Salze.

Die erfindungsgemäßen substituierten 1,8-Naphthyridine der allgemeinen Formel (Ia,b) haben mehrere asymmetrische Kohlenstoffatome und können daher in verschiedenen stereochemischen Formen existieren. Die Erfindung betrifft sowohl die einzelnen Isomeren als auch deren Mischungen.

Je nach der Bedeutung der Gruppe X bzw. des Restes R ergeben sich unterschiedliche Stereoisomere, die im folgenden näher erläutert werden sollten:

a) Steht die Gruppe -X- für eine Gruppe der Formel -CH=CH- , so können die erfindungsgemäßen Verbindungen in zwei stereoisomeren Formen existieren, die an der Doppelbindung E-konfiguriert oder Z-konfiguriert sein können.

Bevorzugt sind diejenigen Verbindungen der allgemeinen Formel (Ia,b) die E-konfiguriert sind.

b) Steht der Rest -R- für eine Gruppe der Formel

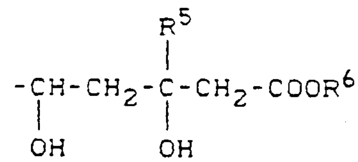

so besitzen die Verbindungen der allgemeinen Formel (Ia,b) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration oder in der threo-Konfiguration vorliegen.

Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form).

Bevorzugt sind hierbei die Erythto-konfigurierten Isomeren, besonders bevorzugt das 3R,5S-Isomere sowie das 3R,5S-3S,5R-Racemat.

c) Steht der Rest -R- für eine Gruppe der Formel

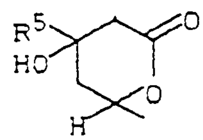

so besitzen die substituierten 1,8-Naphthyridine mindestens zwei asymmetrische Kohlenstoffatome, nämlich das Kohlenstoffatom an dem die Hydroxygruppe gebunden ist, und das Kohlenstoffatom an welchem der Rest der Formel

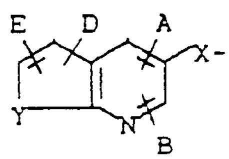

gebunden ist. Je nach der Stellung der Hydroxygruppe zur freien Valenz am Lactonring können die substituierten 1,8-Naphthyridine als cis-Lactone oder als trans-Lactone vorliegen.

Sowohl vom cis-Lacton aus als auch vom trans-Lacton existieren wiederum jeweils zwei Isomere nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomere sind die trans-Lactone. Besonders bevorzugt ist hierbei das 4R,6S-Isomere (trans) sowie das 4R,6S-4S,6R-Racemat.

Außerdem wurde ein Verfahren zur Herstellung der substituierten 1,8-Naphthyridine der allgemeinen Formel (Ia,b) gefunden, das dadurch gekennzeichnet ist, daß man Ketone der allgemeinen Formel (VIII)

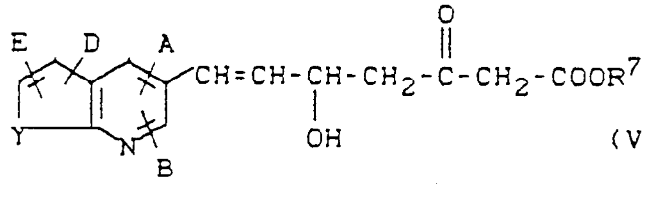
(VIII)

in welcher

A, B, D, E und Y die oben angegebene Bedeutung haben, und

$R^7$ - für Alkyl steht,

reduziert,

im Fall der Herstellung der Säuren die Ester verseift,

im Fall der Herstellung der Lactone die Carbonsäuren cyclisiert,

im Fall der Herstellung der Salze entweder die Ester oder die Lactone verseift,
im Fall der Herstellung der Ethylenverbindungen (X = $-CH_2-CH_2-$) die Ethenverbindungen (X = -CH=CH-) nach üblichen Methoden hydriert,
und gegebenenfalls Isomeren trennt.

Das erfindungsgemäße Verfahren kann durch das folgende Formelschema beispielhaft erläutert werden:

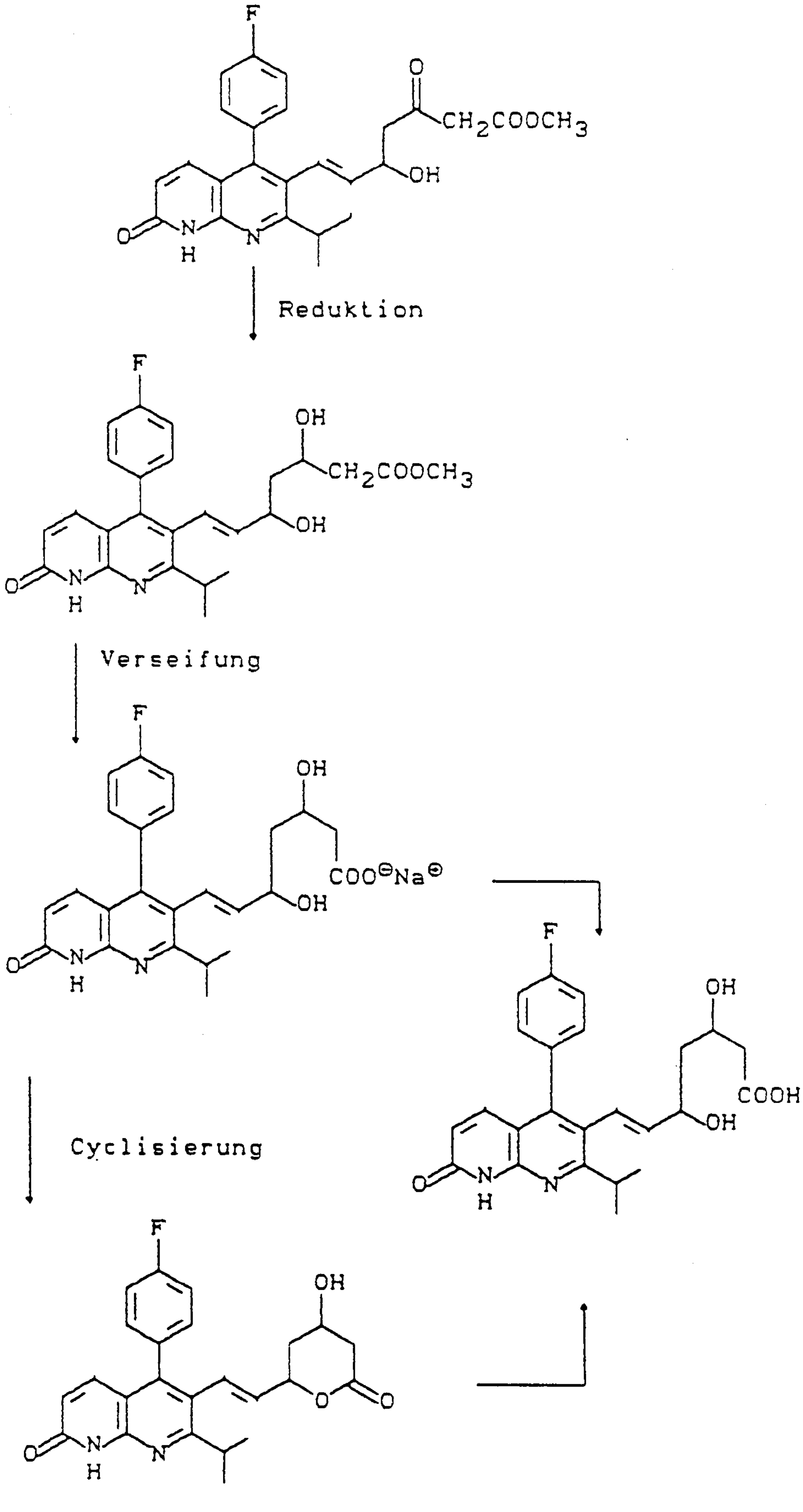


Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-borate, Natrium-trialkylhydrido-boranaten, Natrium-cyano-trihydrido-boranat oder Lithiumaluminiumhydrid durchgeführt. Ganz besonders bevorzugt wird die Reduktion mit Natriumhorhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispielsweise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80°C bis +30°C, bevorzugt von -78°C bis 0°C.

Das erfindungesgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Metoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (Ia,b) in welchen X für eine Ethylengruppierung steht, kann die Reduktion der Ketone (III) unter solchen Bedingungen durchgeführt werden, unter denen sowohl die Carbonylgruppe als auch die Doppelbindung reduziert wird.

Außerdem wurde ein Verfahren zur Herstellung der substituierten 1,8-Naphthyridine der allgemeinen Formel (Ia,b) gefunden, das dadurch gekennzeichnet ist, daß man Ketone der allgemeinen Formel (VIII)

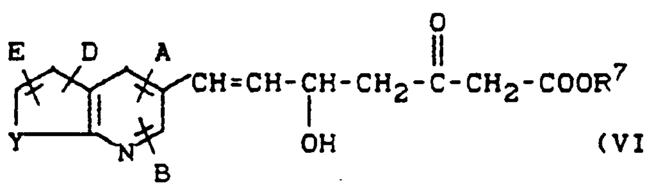

(VIII)

in welcher

A, B, D, E und Y die oben angegebene Bedeutung haben, und

$R^7$ - für Alkyl steht,

reduziert,

im Fall der Herstellung der Säuren die Ester verseift,

im Fall der Herstellung der Lactone die Carbonsäuren cyclisiert,

im Fall der Herstellung der Salze entweder die Ester oder die Lactone verseift,

im Fall der Herstellung der Ethylenverbindungen (X = $-CH_2-CH_2-$) die Ethenverbindungen (X = -CH=CH-) nach üblichen Methoden hydriert,

und gegebenenfalls Isomeren trennt.

Das erfindungsgemäße Verfahren kann durch das folgende Formelschema beispielhaft erläutert werden:

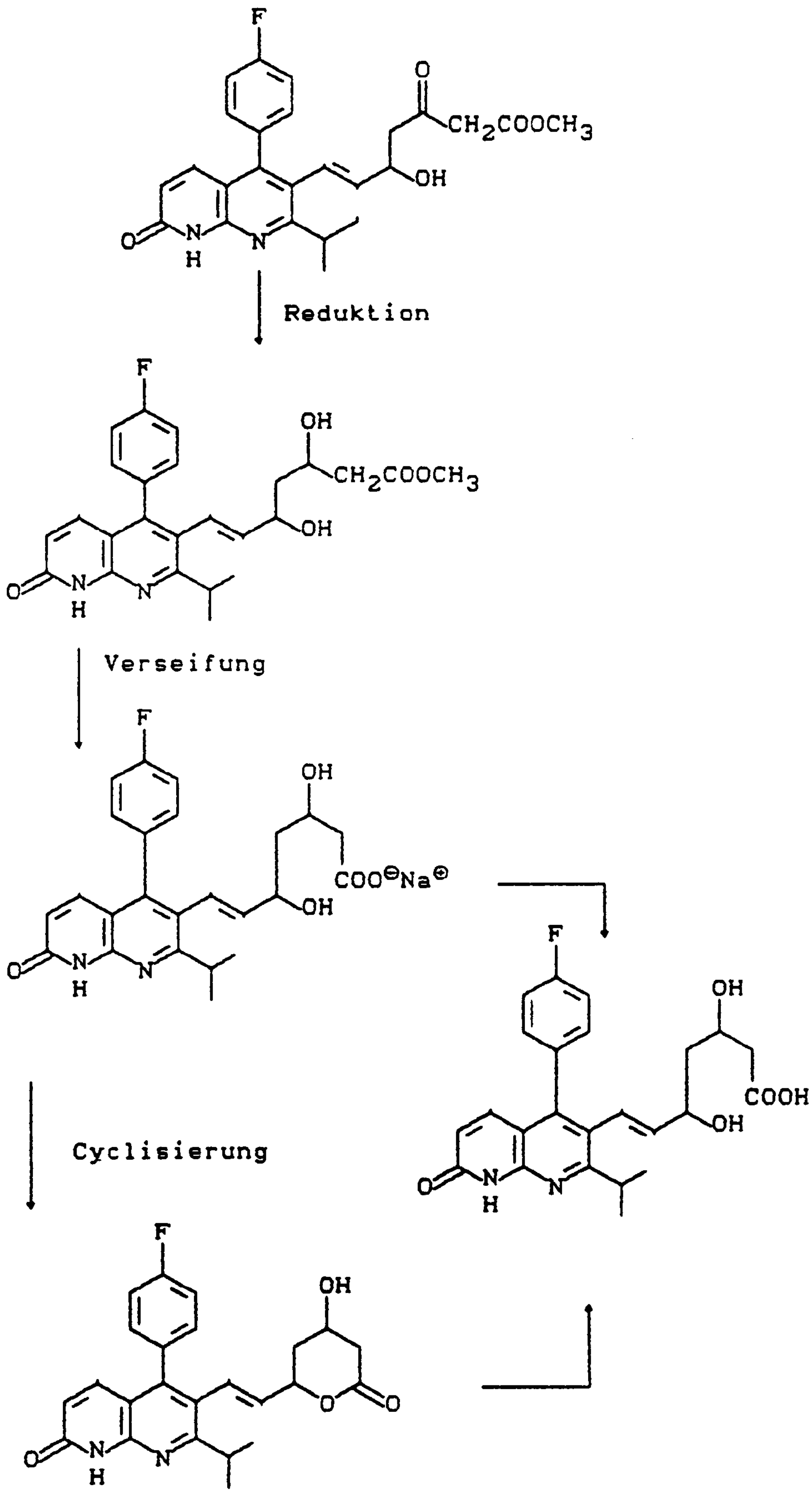


Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-borate, Natrium-trialkyl-hydrido-boranaten, Natrium-cyano-trihydrido-boranat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispielsweise die Alkoxycarbonylgruppe nicht verändert werden Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80°C bis +30°C, bevorzugt von -78°C bis 0° C.

Das erfindungesgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen X für eine Ethylengruppierung steht, kann die Reduktion der Ketone (III) unter solchen Bedingungen durchgeführt werden, unter denen sowohl die Carbonylgruppe als auch die Doppelbindung reduziert wird.

Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ic)

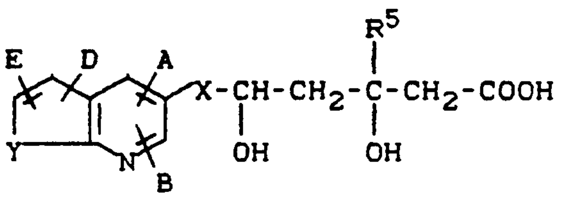

(Ic)

in welcher
A, B, D, E, Y und $R^5$ die oben angegebene Bedeutung haben.

Die Carbonsäureester im Rahmen der allgemeinen Formel (I) entsprechen der Formel (Id)

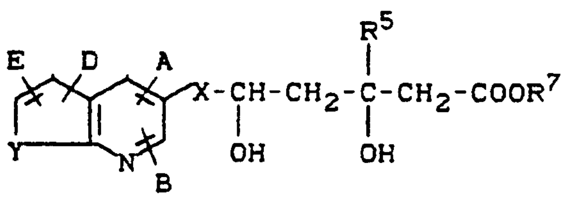

(Id)

in welcher
A, B, D, E, Y und $R^5$ die oben angegebene Bedeutung haben,
und
$R^7$ - für Alkyl steht.

Die Salze der erfindungsgemäßen Verbindungen im Rahmen der allgemeinen Formel (I) entsprechen der Formel (Ie)

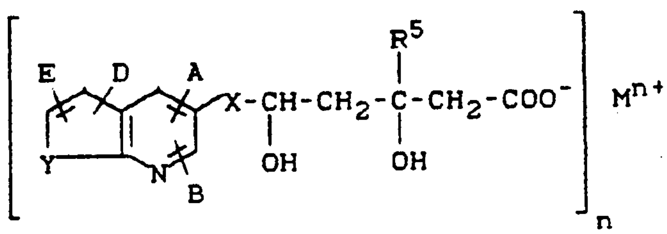

(Ie)

in welcher
A, B, D, E, Y und $R^5$ die oben angegebene Bedeutung haben,
und
$M^{n+}$ für ein Kation steht, wobei n die Wertigkeit angibt.

Die Lactone im Rahmen der allgemeinen Formel (I) entsprechen der Formel (If)

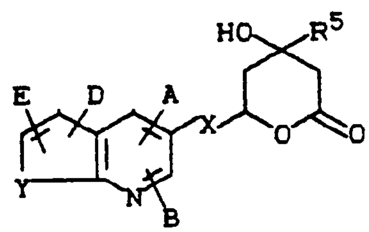

(If)

in welcher
A, B, D, E, Y und $R^5$ die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Wasser, Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Methanol, Tetrahydrofuran oder Wasser verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäuren. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40°C bis +200°C, bevorzugt von -25°C bis +50°C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N'-Dicyclohexylcarbodiimid-Paratoluolsulfonat, N-Cyclohexyl-N'-[2-(N''-methylmorpholinium)ethyl]carbodiimid oder N-(3-Dimethylaminopropyl)-N'-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Ganz besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0°C bis +80°C, bevorzugt von +10°C bis +50°C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Ester. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ig)

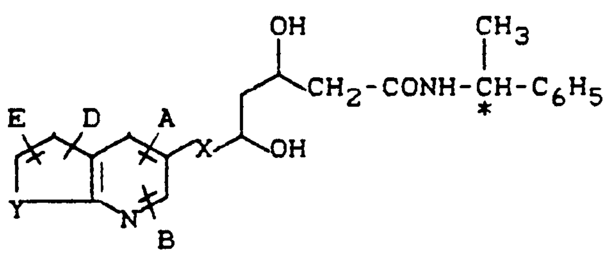

(Ig)

überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kaliumhydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden:

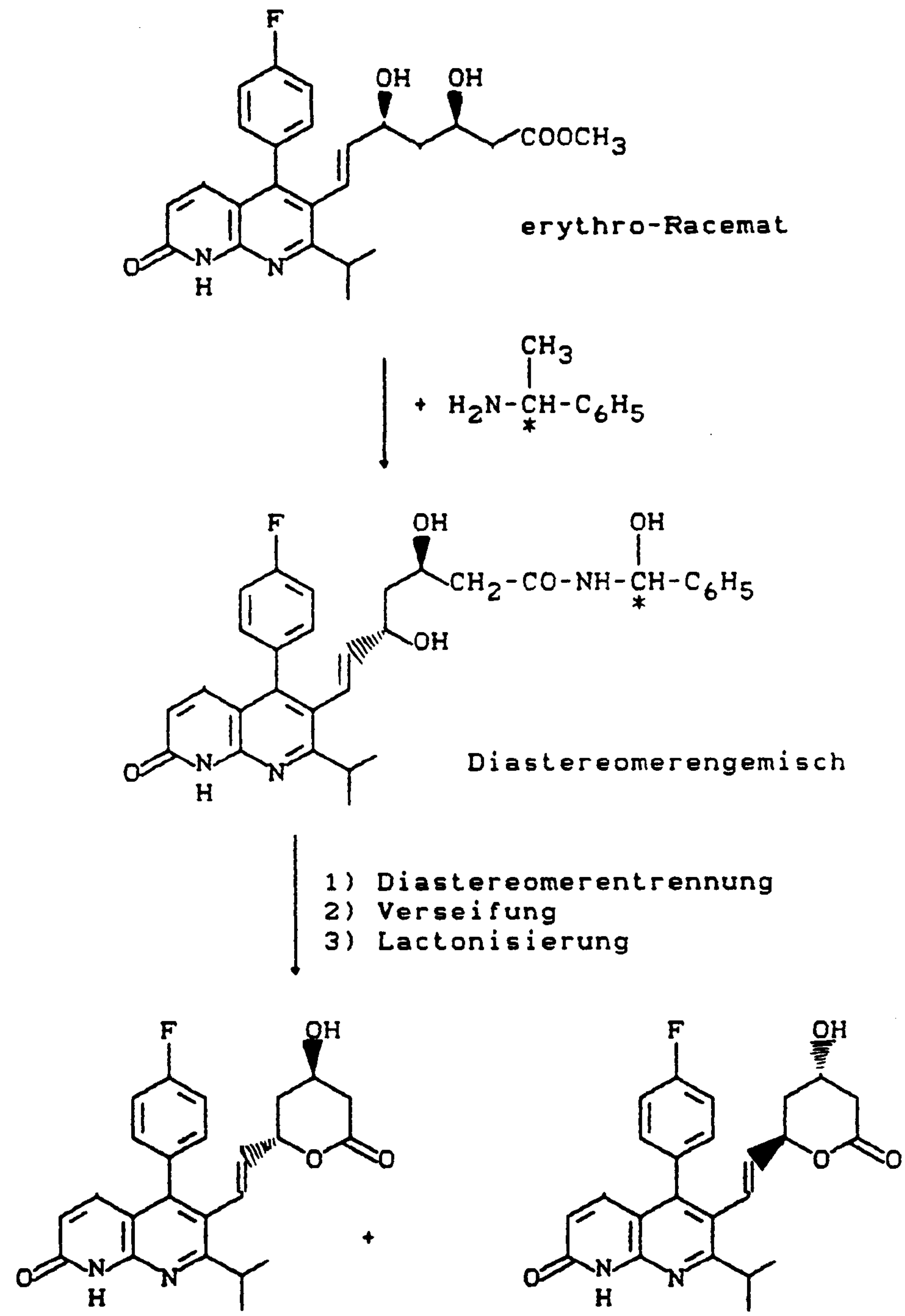


Die als Ausgangsstoffe eingesetzten Ketone (VIII) sind neu.

Es wurde ein Verfahren zur Herstellung der erfindungsgemäßen Ketone der allgemeinen Formel (VIII)

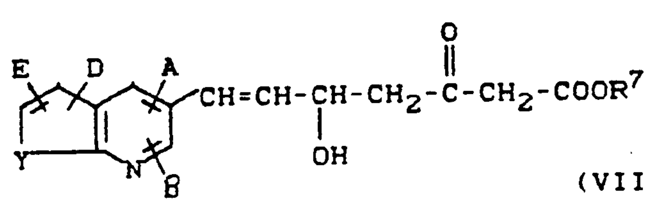

(VIII)

in welcher

A, B, D, E, Y und $R^7$ die oben angegebene Bedeutung haben,
gefunden, das dadurch gekennzeichnet ist, daß man
Aldehyde der allgemeinen Formel (IX)

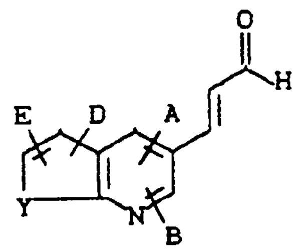

(IX)

in welcher
A, B, D, E und Y die oben angegebene Bedeutung haben,
in inerten Lösemitteln mit Acetessigester der allgemeinen Formel (X)

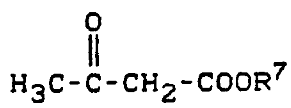

(X)

in welcher
$R^7$ die oben angegebene Bedeutung hat,
in Anwesenheit von Basen umsetzt.

Das erfindungsgemäße Verfahren kann beispielsweise durch folgendes Formelschema erläutert werden:

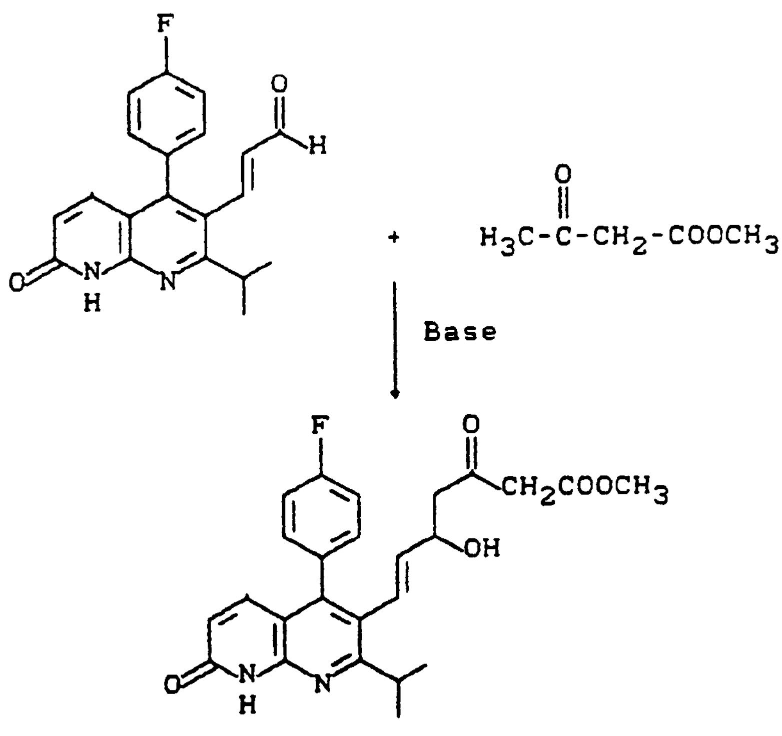


Als Basen kommen hierbei die üblichen stark basischen Verbindungen in Frage. Hierzu gehören bevorzugt lithiumorganische Verbindungen wie beispielsweise N-Butyllithium, sec.Butyllithium, tert.Butyllithium oder Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid

oder deren Gemisch eingesetzt.

Eventuell sind Zusätze von Metallhalogeniden wie z.B. Magnesiumchlorid, Zinkchlorid oder Zinkbromid vorteilhaft. Besonders bevorzugt ist der Zusatz von Zinkhalogeniden.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erdölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80°C bis +50°C, bevorzugt von -20°C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 5, bevorzugt von 1 bis 3 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt:
Acetessigsäuremethylester, Acetessigsäureethylester, Acetessigsäurepropylester, Acetessigsäureisopropylester.

Die Herstellung der als Ausgangsstoffe eingesetzten Aldehyde der allgemeinen Formel (IX) soll im folgenden beispielhaft für die 1,8-Naphthyridine des Typs (Ia) erläutert werden

[A]

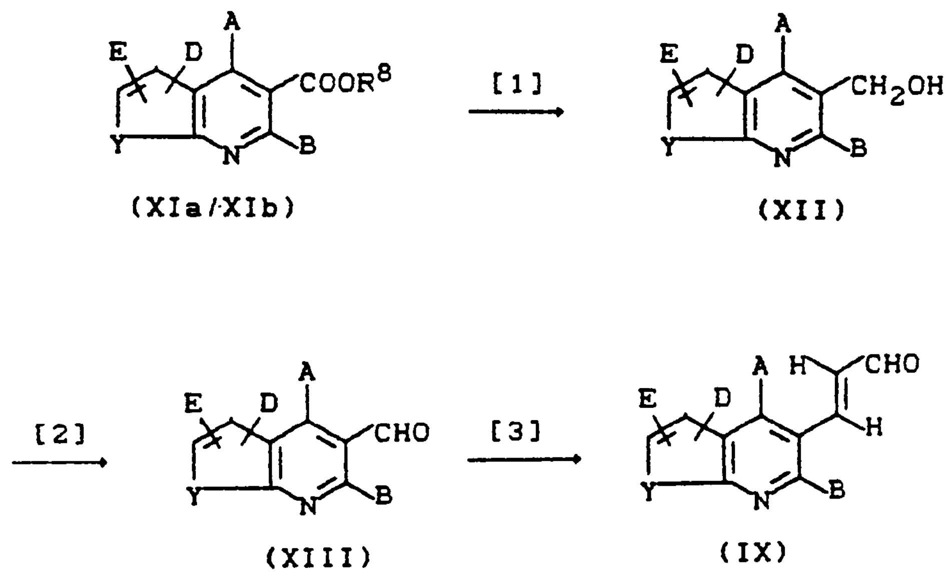


(XIa/XIb) (XII) (XIII) (IX)

Hierbei werden gemäß Schema A 1,8-Naphthyridine der Formel (XI), in welchen $R^8$ für einen Alkylrest mit bis zu 4 Kohlenstoffatomen steht, im ersten Schritt [1] in inerten Lösemitteln wie Ethern, beispielsweise Diethylether, Tetrahydrofuran oder Dioxan oder Kohlenwasserstoffe wie Toluol, vorzugsweise Toluol, mit Metallhydriden als Reduktionsmittel, beispielsweise Lithiumaluminiumhydrid, Natriumcyanoborhydrid, Natriumaluminiumhydrid, Diisobutylaluminiumhydrid oder Natriumbis-(2-methoxyethoxy)-dihydroaluminat, in Temperaturbereichen von -75°C bis +100°C, vorzugsweise von -80°C bis Raumtemperatur, bzw. von Raumtemperatur bis -78°C je nach verwendetem Reduktionsmittel zu den Hydroxymethylverbindungen (XII) reduziert. Vorzugsweise wird die Reduktion mit Diisobutylaluminiumhydrid in Tetrahydrofuran oder Toluol in einem Temperaturbereich von -78°C bis Raumtemperatur durchgeführt. Die Hydroxymethylverbindungen (XII) werden im zweiten Schritt [2] nach üblichen Methoden zu den Aldehyden (XIII) oxidiert. Die Oxidation kann beispielsweise mit Pyridiniumchlorochromat, gegebenenfalls in Anwesenheit von Aluminiumoxid, in inerten Lösemitteln wie Chlorkohlenwasserstoffen, vorzugsweise Methylenchlorid, in einem Temperaturbereich von 0°C bis 60°C, bevorzugt bei Raumtemperatur durchgeführt werden, oder aber mit Dimethylsulfoxid nach den üblichen Methoden der Swern Oxidation durchgeführt werden. Die Aldehyde (XIII) werden im dritten Schritt [3] mit Diethyl-2-(cyclohexylamino)-vinylphosphonat in Anwesenheit von Natriumhydrid in inerten Lösemitteln wie Ethern, beispielsweise Diethylether, Tetrahydrofuran oder Dioxan, vorzugsweise in Tetrahydrofuran, in einem Temperaturbereich von -20°C bis +40°C, vorzugsweise von -5°C bis Raumtemperatur zu den Aldehyden (IX) umgesetzt.

Die hierbei als Ausgangsstoffe eingesetzten 1,8-Naphthyridine der Formel (XI) sind neu.

Verbindungen der Formel (XIa), in welcher Y für die Gruppe der Formel

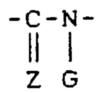

steht
worin
G und Z die oben angegebene Bedeutung haben, können erhalten werden, indem man gemäß Schema [B] 3,4-Dihydro-1,8-naphthyridine der Formel (XIV), in welcher A, B, D, E, G, Z und $R^8$ die oben angegebene Bedeutung haben, oxidiert. Die Oxidation kann beispielsweise mit Chromoxid oder Natriumnitrit in Eisessig, mit Salpetersäure in wäßriger Suspension, mit Cersalzen wie beispielsweise Ammoniumcernitrat in einem Lösemittel gemisch aus Acetonitril und Wasser, oder mit Dichlordicyano-p-benzochinon in den oben aufgeführten inerten Lösemitteln, in einem Temperaturbereich von -20°C bis +150°C durchgeführt werden.

[B]

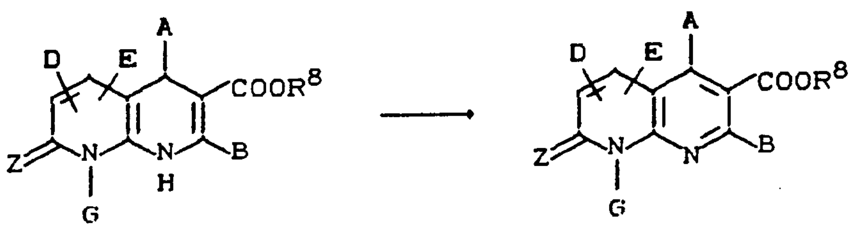

(XIV) (XIa)

Die hierbei als Ausgangsstoffe eingesetzten 3,4-Dihydro-1,8-naphthyridine der allgemeinen Formel (XIV) sind neu.

Man erhält sie gemäß Schema [C] durch Reaktion geeignet substituierter α,β-ungesättigter Carbonsäureester der allgemeinen Formel (XV), worin A, B und $R^8$ die oben angegebene Bedeutung haben, mit substituierten 6-Amino-2-pyridonen der allgemeinen Formel (XVI), in welcher D, E, G und Z die oben angegebene Bedeutung haben.

Die Reaktion kann durch folgendes Formelschema erläutert werden:

[C]

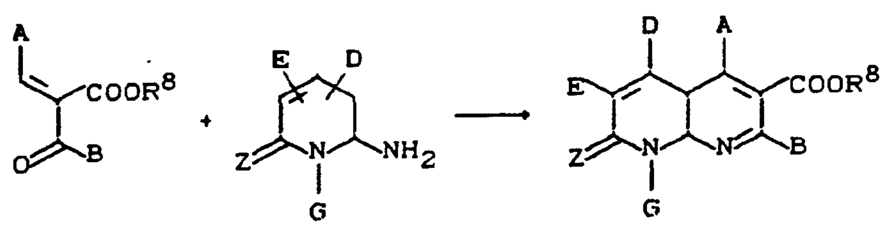

(XV) (XVI) (XIVa)

Das Verfahren kann in Substanz oder in einem hochsiedendem Lösemittel wie beispielsweise Ethylenglykol oder Dimethylformamid, gegebenenfalls in Anwesenheit von Essigsäure bei Raumtemperatur bis +200°C durchgeführt werden. Bevorzugt ist die Umsetzung in Substanz oder Dimethylformamid bei +120°C bis +160°C.

Außerdem können Verbindungen der allgemeinen Formel (I), in welcher Y für die Gruppe der Formel

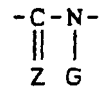

steht,

und G einen $C_1$-$C_6$-Alkylrest bedeutet, hergestellt werden, indem man Verbindungen der allgemeinen Formeln (XIII), (XIV) oder (I), in welchen G für Wasserstoff steht, mit $C_1$-$C_6$-Alkylhalogeniden, wie beispielsweise Methyljodid in Anwesenheit von Basen, wie beispielsweise Kalium-tert.butylat, in den oben erwähnten Lösemitteln, bevorzugt in Dimethylformamid bei Raumtemperatur alkyliert.

Verbindungen der Formel (XIb), in welcher Y für die Gruppe der Formel

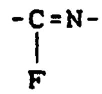

steht,

worin F die oben angegebene Bedeutung hat, können hergestellt werden, indem man Verbindungen der Formel (XIa) nach literaturbekannten Methoden [vgl. L.F. Fieser, M.

Fieser, Reagents for Organic Synthesis, Vol 1, S. 1232 (1967)] entweder mit Trialkyloxoniumsalzen, vorzugsweise mit Trimethyloxoniumtetrafluoroboranat bei Raumtemperatur, oder mit Basen wie beispielsweise Kalium-tert.-butylat oder Natriumhydrid und $C_1$-$C_6$-Alkylhalogeniden wie beispielsweise Methyljodid, Ethyljodid oder Isopropyljodid, bevorzugt mit Isopropyljodid in den oben erwähnten Lösemitteln bei Raumtemperatur umsetzt. Ferner ist es möglich, zunächst mit Phosphorchloriden wie beispielsweise Phosphoroxytrichlorid, in Gegenwart von $C_1$-$C_4$-Dialkylanilinen bei 0°C bis +100°C eine Chlorierungsreaktion durchzuführen und anschließend durch nucleophile Substitution die Gruppe F einzuführen.

Die erfindungsgemäßen Verbindungen der allgemeinen Formel (I) besitzen wertvolle pharmakologische Eigenschaften und können in Arzneimitteln eingesetzt werden. Insbesondere sind sie Inhibitoren der 3-Hydroxy-3-methylglutaryl-Coenzym A (HMG-CoA) Reduktase und infolge dessen Inhibitoren der Cholesterolbiosynthese. Sie können deshalb zur Behandlung von Hyperlipoproteinämie, Lipoproteinämie oder Atherosklerose eingesetzt werden. Die erfindungsgemäßen Wirkstoffe bewirken außerdem eine Senkung des Cholesteringehaltes im Blut.

Die Enzymaktivitätsbestimmung wurde modifiziert nach G.C. Ness et al, Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Cholestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m $K_xH_y$ Phosphat, 0,03 m Ethylendiamintetraessigsäure, 0,002 m Dithiothreit (SPE)-Puffer pH 7,2, homogenisiert. Anschließend wurde 15 Minuten bei 15 000* g zentrifugiert und das Sediment verworfen. Der Überstand wurde 75 Minuten bei 100 000 g sedimentiert. Das Pellet wird in 1/4 Volumen SPE-Puffer aufgenommen, nochmals homogenisiert und anschließend erneut 60 Minuten bei 100.000 zentrifugiert. Das Pellet wird mit der 5-fachen Menge ihres Volumes SPE-Puffer aufgenommen, homogenisiert und bei -78°C eingefroren und gelagert (= Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37°C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase 35 µMol $K_xH_y$-Phosphat pH 7,2, 20 µl Enzympräparation und 56 µMol 3-Hydroxy-3-methyl-glutaryl Coenzym A (Glutaryl-3-$^{14}$C) 100 000 dpm.

Nach Inkubation von 60 Minuten bei 37°C wurde der Ansatz zentrifugiert und 600 µl des Überstandes auf eine 0,7 x 4 cm mit einem 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. $IC_{50}$-Werte wurden durch Auftrag der prozentualen Hemmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 1 gesetzt und mit dem simultan bestimmten $IC_{50}$-Wert der Testverbindung verglichen.

Die neuen Wirkstoffe können in bekannter Weise in die üblichen Formulierungen überführt werden, wie

Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthaltend. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchem die Verabreichung erfolgt.

So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

## Herstellungsbeispiele

### Beispiel 1

E,Z-2-Ethoxycarbonyl-1-(4-fluorphenyl)-4-methyl-pent-1-en-3-on

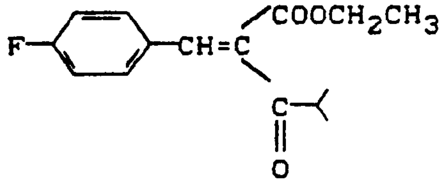

Zu 554 g (3,5 mol) Isobutyrylessigsäureethylester und 434 g (3,5 mol) 4-Fluorbenzaldehyd in 1,8 l Isopropanol gibt man eine Lösung von 20 ml (0,2 mol) Piperidin und 12 ml (0,21 mmol) Essigsäure in 200 ml Isopropanol. Man rührt 1 Tag bei Raumtemperatur, engt im Vakuum ein und destilliert den Rückstand im Hochvakuum.
Ausbeute: 796 g (86% der Theorie) gelbliches Öl
Kp.: 135 - 140°C (0.2 mbar)

Beispiel 2

6-Ethoxycarbonyl-5-(4-fluorphenyl)-1,2-dihydro-7-isopropyl-2-oxo-1,8-naphthyridin

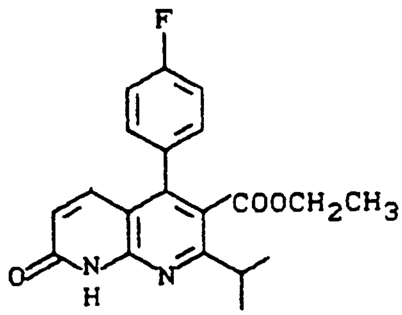

66 g (0,6 mol) 6-Amino-pyridin-2-on [O.A. Seide, A.I. Titow, Ber. dtsch. Chem. Ges. 69, 1884 (1936)] und 159 g (0,6 mol) der Verbindung aus Beispiel 1 werden in 50 ml Dimethylformamid 3 h bei 100°C gerührt. Dann werden alle flüchtigen Bestandteile bis zu einer Badtemperatur von 200°C und einem Druck von 0.3 mbar abdestilliert. Der dunkle Rückstand wird mit Chloroform und Chloroform/Methanol (10:1) über 1 kg Kieselgel filtriert. Nach Abziehen der Lösemittel bleibt ein brauner Schaum (60 g) zurück.
Dieses Rohprodukt löst man in 0.7 l Dichlormethan, versetzt mit 39,7 g (0,175 mol) Dichlor-dicyano-p-benzochinon und rührt 1 h bei Raumtemperatur. Nach Absaugen der Reaktionsmischung und Einengen des Filtrats bleibt ein Rückstand, der mit Petrolether/Essigester (5:1) bis (2:1) über 1 kg Kieselgel filtriert wird. Den aus dem Filtrat erhaltenen Rückstand kristallisiert man aus Essigester/Ether um.
Ausbeute: 21,7 g (10% der Theorie) farblose Kristalle
Schmp.: 182°C

Beispiel 3

5-(4-Fluorphenyl)-1,2-dihydro-6-hydroxy-methyl-7-isopropyl-2-oxo-1,8-naphthyridin

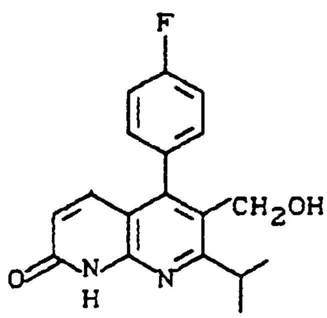

Zu einer Lösung von 21,7 g (61 mmol) der Verbindung aus Beispiel 2 in 700 ml wasserfreiem Toluol tropft man bei -78°C während 2 h 200 ml einer 1,5 molaren Lösung von Diisobutylaluminiumhydrid in Toluol zu und rührt eine weitere Stunde bei dieser Temperatur. Man läßt langsam auf Raumtemperatur erwärmen, währenddessen man vorsichtig 200 ml Wasser zutropft. Die Mischung wird über Kieselgur abgesaugt, mit Essigester nachgewaschen und das Kieselgur nochmals mit Essigester ausgekocht. Die organische Phase wird mit Kochsalzlösung gewaschen, über Natriumsulfat getrocknet, zur Trockne eingeengt und der Rückstand aus Essigester/Ether kristallisiert.
Ausbeute: 18,8 g (97% der Theorie) farblose Kristalle
Schmp.: 230°C (aus Essigester)

Beispiel 4

5-(4-Fluorphenyl)-6-formyl-1,2-dihydro-7-isopropyl-2-oxo-1,8-naphthyridin

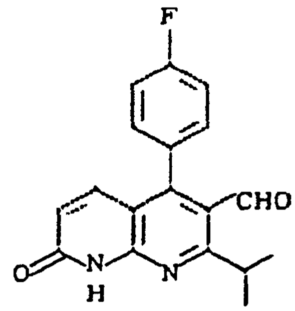

Zu einer Lösung von 18,8 g (60 mmol) der Verbindung aus Beispiel 3 in 300 ml Tetrahydrofuran gibt man 12,3 g Aluminiumoxid und 25,9 g (120 mmol) Pyridiniumchlorochromat und rührt 1 h bei Raumtemperatur. Man filtriert über 500 g Kieselgel und wäscht mit je 1 l Dichlormethan, Petrolether/Essigester (1:1) und Essigester nach. Das einrotierte Filtrat wird aus Methanol kristallisiert.
Ausbeute: 13,3 g (71% der Theorie) farblose Kristalle
Schmp.: 223°C

Beispiel 5

(E)-3-[5-(4-Fluorphenyl)-1,2-dihydro-7-isopropyl-2-oxo-1,8-naphthyridin-6-yl]-prop-2-enal

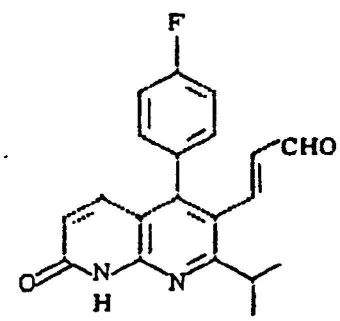

Zu einer Suspension von 3,0 g (100 mmol) 80%igem Natriumhydrid in 80 ml wasserfreiem Tetrahydrofuran tropft man bei 0°C unter Argon eine Lösung von 13,1 g (50 mmol) Diethyl-2-(cyclohexylamino)-vinyl-phosphonat in 80 ml Tetrahydrofuran und rührt 30 min bei dieser Temperatur. Dann wird eine Lösung von 13,0 g (42 mmol) der Verbindung aus Beispiel 4 in 80 ml Tetrahydrofuran bei 0°C - 5°C zugetropft und anschließend 1 h unter Rückfluß gekocht. Man versetzt vorsichtig mit 200 ml Wasser und extrahiert dreimal mit Essigester. Nach Einengen der organischen Phasen wird der Rückstand mit einer Mischung aus 500 ml Toluol, 500 ml Wasser und 27,5 g (218 mmol) Oxalsäuredihydrat 2 h unter Rückfluß gekocht. Die Toluolphase wird eingeengt und der Rückstand mit Essigester behandelte. Dabei fallen 11,35 g (80% der Theorie) farblose Kristalle vom Schmp. 261°C an.

Beispiel 6

Methyl-erythro-(E)-7-[5-(4-fluorphenyl)-1,2-dihydro-7-isopropyl-2-oxo-1,8-naphthyridin-6-yl]-3,5-dihydroxyhept-6-enoat

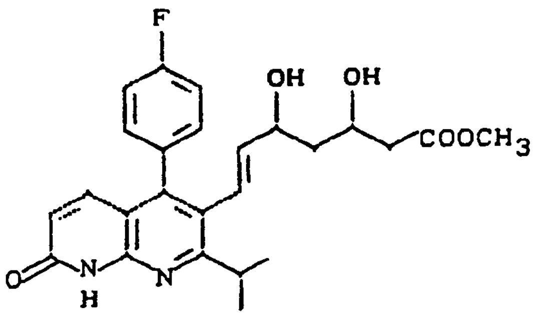

Zu einer Suspension von 1,69 g (56,4 mmol) 80%igem Natriumhydrid in 50 ml wasserfreiem Tetrahydrofuran tropft man bei -5°C bis 0°C unter Argon 5,47 g (50.7 mmol) Acetessigsäuremethylester in 5 ml Tetrahydrofuran. Nach 15 min werden bei derselben Temperatur 41 ml (67,6 mmol) 15%iges Butyllithium in Hexan zugetropft und nach weiteren 15 min eine Lösung von 5,68 g (16,9 mmol) der Verbindung aus Beispiel 5 in 150 ml Tetrahydrofuran. Man rührt eine Stunde bei Raumtemperatur, tropft dann 11,2 g Essigsäure in 120 ml Wasser vorsichtig zu und extrahiert dreimal mit Essigester. Die organischen Phasen werden mit gesättigter Natriumhydrogencarbonat- und Kochsalzlösung gewaschen, über Natriumsulfat getrocknet und eingeengt. Man erhält 7,8 g rohes Methyl-(E)-7-[5-(4-fluorphenyl)-1,2-dihydro-7-isopropyl-2-oxo-1,8-naphthyridin-6-yl]-5-hydroxy-3-oxo-hept-6-enoat, als oranges Öl.
Dieses Rohprodukt wird unter Argon in 100 ml wasserfreiem Tetrahydrofuran gelöst, 20,3 ml einer 1 M Lösung von Triethylboran in Tetrahydrofuran zugesetzt und 5 min Luft durch die Lösung geleitet. Man kühlt auf -78°C ab, gibt 767 mg (20,3 mmol) Natriumborhydrid zu, tropft dann langsam 11 ml Methanol zu und rührt eine weitere Stunde bei -78°C bis -75°C. Man läßt dann auf Raumtemperatur erwärmen, wobei ab ca. -30°C 53 ml 30%iges Wasserstoffperoxid und 50 ml Wasser zugetropft werden. Es wird dreimal mit Essigester extrahiert, die organischen Phasen über Natriumsulfat getrocknet und eingeengt. Der Rückstand wird an 120 g Kieselgel (230 - 400 mesh) mit Essigester/Petrolether (2:1) bis (4:1) chromatographiert. Man erhält einen farblosen Schaum (4,09 g), der aus Methanol/Wasser umkristallisiert wird.
Ausbeute: 2,32 g (30% der Theorie) farblose Kristalle
Schmp.: 148°C

Beispiel 7

Erythro-(E)-7-[5-(4-fluorphenyl)-1,2-dihydro-7-isopropyl-2-oxo-1,8-naphthyridin-6-yl]-3,5-dihydroxy-hept-6-en-carbonsäure-Natriumsalz

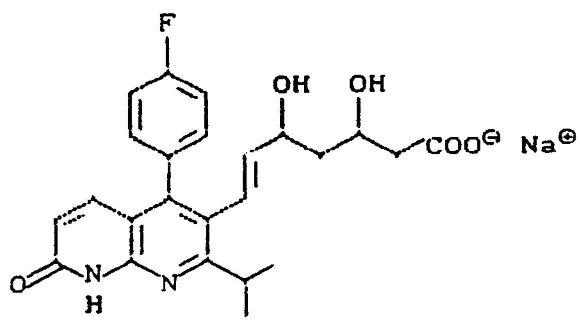

100 mg (0,22 mol) der Verbindung aus Beispiel 6 werden in 2,2 ml Tetrahydrofuran und 2,2 ml 0,1 N Natronlauge 1 h bei Raumtemperatur gerührt. Man zieht die Lösemittel ab und trocknet über Phosphorpentoxid in Hochvakuum.
Ausbeute: 85 mg farblose Kristalle
Schmp.: ab 170°C (Zers.)

Beispiel 8

Methyl-erythro-(E)-7-[5-(4-fluorphenyl)-1,2-dihydro-7-isopropyl-1-methyl-2-oxo-1,8-naphthyridin-6-yl]-3,5-dihydroxy-hept-6-enoat

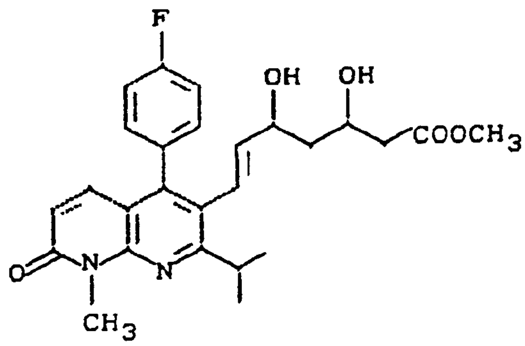

Zu einer Lösung von 69 mg (0,6 mmol) Kalium-tert.butylat in 5 ml Dimethylformamid gibt man eine Lösung von 275 mg (0,6 mol) der Verbindung aus Beispiel 6 in 5 ml Dimethylformamid und 86 mg (0,6 mol) Methyljodid und rührt 4 h bei Raumtemperatur. Dann werden weitere 138 mg (1,2 mmol) Kalium-tert.butylat und 344 mg (2,4 mmol) Methyljodid zugegeben und über Nacht gerührt. Der Ansatz wird auf Wasser gegossen, dreimal mit Essigester extrahiert, die organische Phase getrocknet und eingeengt (0,28 g gelbes Öl). Säulenchromatographie an 20 g Kieselgel (230-400 mesh) mit Petrolether/Essigester (1:1) und Essigester ergibt 52 mg (18% der Theorie) eines farblosen Öls.

$^1$H-NMR ($CDCl_3$): $\delta$ = 1,25 - 1,45 (m, 8H, CH($\underline{CH_3}$)$_2$ + 4-H); 2,45 (m, 2H, 2-H); 3,07 (d, 1H, OH); 3,45 (sept, 1H, $\underline{CH}$($CH_3$)$_2$); 3,53 (d, 1H, OH) 3,72 (s, 3H; O-$CH_3$); 3,9 (s, 3H, N-$CH_3$); 4,1 (m, 1H, C-H); 4,35 (m, 1H, C-H); 5,3 (dd, 1H, 6-H); 6,43 (d, 1H, 7-H); 6,6 (d, 1H, 3'-H); 7,13 (m, 4H, Aromaten-H); 7,28 (d, 1H, 4'-H) ppm.

Beispiel 9

(E)-3-[1-Ethyl-5-(4-fluorphenyl)-1,2-dihydro-7-isopropyl-2-oxo-1,8-naphthyridin-6-yl]-prop-2-enal

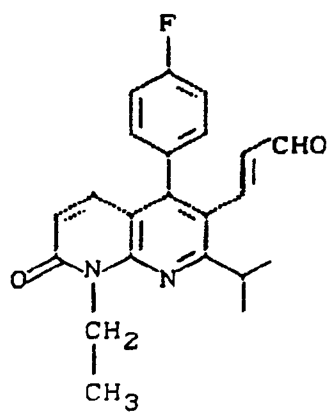

1,0 g (3 mmol) der Verbindung aus Beispiel 5 werden in 20 ml Dimethylformamid suspendiert und nacheinander mit einer Lösung von 366 mg (3,3 mmol) Kalium-tert.-butylat in 5 ml Dimethylformamid und 510 mg (3,3 mmol) Ethyliodid in 1 ml Dimethylformamid tropfenweise versetzt. Man rührt 90 min bei 60°C, gießt auf 150 ml Eiswasser und extrahiert dreimal mit Essigester. Die organischen Phasen werden getrocknet, eingeengt und der Rückstand an 30 g Kieselgel (230 - 400 mesh, Säulendurchmesser 2,5 cm) mit Petrolether/Essigester (5:1) und (3:1) chromatographiert.

Ausbeute: 850 mg (78 %), farblose Kristalle vom Schmelzpunkt: 123°C

In Analogie zu Beispiel 9 werden aus der Ausgangsverbindung des Beispiels 5 hergestellt:

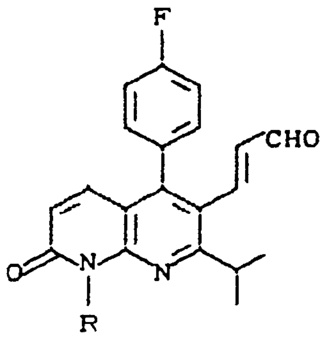

| Bsp. Nr. | R | Reagenz | F (°C) | Ausbeute |
|---|---|---|---|---|
| 10 | $CH_2$ 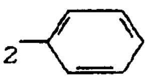 | Br-$CH_2$ 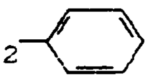 | 164 | 83 % |
| 11 | 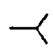 | 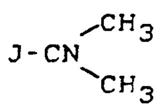 | | |

Beispiel 12

Methyl-erythro-(E)-7-[1-ethyl-5-(4-fluorphenyl)-1,2-dihydro-7-isopropyl-2-oxo-1,8-naphthyridin-6-yl]-3,5-dihydroxy-hept-6-enoat

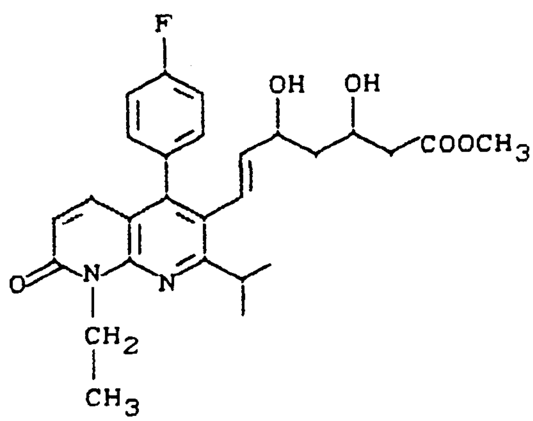

830 mg (2,3 mmol) der Verbindung aus Beispiel 9 werden analog der Vorschrift für Beispiel 6 umgesetzt, wobei 103 mg (3,4 mmol) Natriumhydrid, 0,27 ml (2,5 mmol) Acetessigester und 2,79 ml (4,6 mmol) 15 % Butyllithium verwendet werden.

Ausbeute: 155 mg (14 %)

farblose Kristalle, Schmelzpunkt: 110°C

(aus Ether, Petrolether kristallisiert).

In Analogie zu Beispiel 12 werden hergestellt:

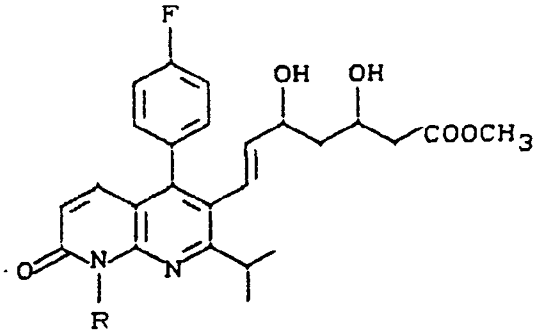

| Bsp. Nr. | R | Ausgangs-verbindung Beispiel | F (°C) | $^1$H-NMR (COCl$_3$) |
|---|---|---|---|---|
| 13 | 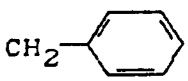 | 10 | amorph | 2,45 (m,2H)<br>3,4 (m,1H)<br>3,7 (s,3H)<br>4,1 (m,1H)<br>4,35 (n,1H)<br>5,3 (dd,1H)<br>5,8 (S,2H)<br>6,4 (d,1H)<br>6,6 (d,4H) |
| 14 | 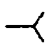 | 11 | | |

Beispiel 15

Erythro-(E)-7-[1-ethyl-5-(4-fluorphenyl)-1,2-dihydro-7-isopropyl-2-oxo-1,9-naphthyridin-6-yl]3,5-dihydroxy-hept-6-en-carbonsäure-Natriumsalz

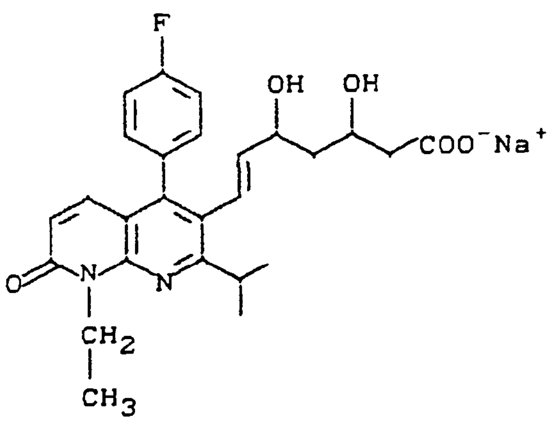

100 mg (0,2 mmol) der Verbindung aus Beispiel 12 werden analog der Vorschrift für Beispiel 7 umgesetzt.
Ausbeute: 82 mg (81 %), farbloser Schaum
FAB-MS: 513 (100 %, M + Na + H, 491/20 %, M + H)

Beispiel 16

trans-6-{2-[5-(4-Fluorphenyl)-7-isopropyl-2-oxo-1,2-dihydro-1,8-naphthyridin-6-yl]-ethenyl}-4-hydroxy-3,4,5,6-tetrahydro-2H-pyran-2-on

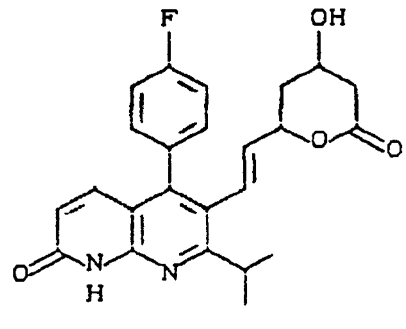

925 mg (2 mmol) der Verbindung aus Beispiel 7 werden in 20 ml THF und 20 ml Wasser gelöst, mit 1 N HCl bei pH-Wert 5 eingestellt, mit 384 mg (2 mmol) N-(3-Dimethylaminopropyl)-N'-ethylcarbodiimid versetzt und 1 Tag bei Raumtemperatur gerührt. Man gibt weitere 192 g (1 mmol) des Carbodiimids zu und rührt noch 2 Tage. Die Extraktion erfolgt mit Essigester; das Trocknen der organischen Phase und Chromatographie erfolgen an 30 g Kieselgel (230 bis 400 mesh) mit Chloroform, Chloroform-Methanol (5:1), und die Kristallisation aus Chloroform/Ether/Petrolether ergibt 179 mg (21 %) farblose Kristalle vom Schmp.: 233°C.

Beispiel 17

trans-6-{2-[1-Benzyl-5-(4-fluorphenyl)-7-isopropyl-2-oxo-1,2-dihydro-1,8-naphthyridin-6-yl]-ethenyl}-4-hydroxy-3,4,5,6-tetrahydro-2H-pyran-2-on

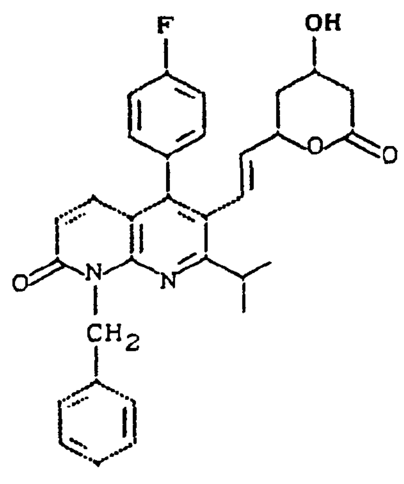

Zu einer Suspension von 250 mg (0,59 mmol) der Verbindung aus Beispiel 16 in 5 ml DMF gibt man eine Lösung von 73 mg (0,65 mmol) Kalium-tert.-butylat in 1 ml DMF und eine Lösung von 110 mg (0,65 mmol) Benzylbromid in 1 ml DMF und rührt 2,5 h bei 60°C. Aufarbeitung wie unter Beispiel 8 ergibt 115 mg (38 %) farblosen Schaum.

$^1$H-NMR ($CDCl_3$): $\delta$ = 1,25 (d, 6H); 1,3 - 1,7 (m, 2H); 2,6 (m, 2H); 3,4 (sept, 1H); 4,2 (m, 1H); 5,1 (m, 1H); 5,3 (dd, 3H); 5,8 (s, 2H); 6,5 (d, 1H); 6,6 (d, 1H); 7,0 - 7,3 (m, 8H); 7,5 (m, 2H).

Beispiel 18

trans-6-{2-[1-Cyanomethyl-5-(4-fluorphenyl)-7-isopropyl-2-oxo-1,2-dihydro-1,8-naphthyridin-6-yl]-ethenyl}-4-hydroxy-3,4,5,6-tetrahydro-2H-pyran-2-on

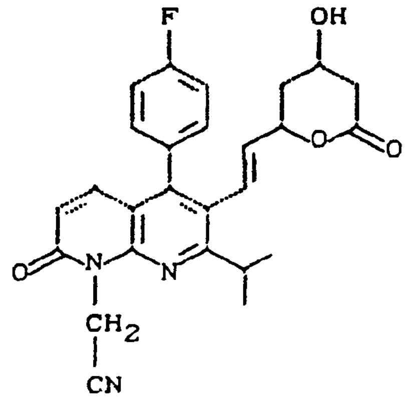

Die Titelverbindung wird analog der Vorschrift für Beispiel 8 aus 120 mg (0,28 mmol) der Verbindung aus Beispiel 7, 57 mg (0,5 mmol) Kalium-tert.-butylat und 38 mg (0,5 mmol) Chloracetonitril hergestellt.
Ausbeute: 68 mg (53 %) beiges Öl.
$^1$H-NMR ($CDCl_3$): $\delta$ = 1,37 (d, 6H); 1,4 - 1,8 (m, 2H); 2,25 (b, 1H); 2,65 (m, 2H); 3,45 (sept, 1H); 4,25 (m, 1H); 5,15 (m, 1H); 5,38 (dd, 1H); 5,47 (s, 2H); 6,03 (d, 1H); 6,12 (d, 1H); 7,13 (m, 4H); 7,38 (d, 1H).

Beispiel 19

(E)-3-[2-Chlor-5-(4-fluorphenyl)-7-isopropyl-1,8-naphthyridin-6-y]-prop-2-enal

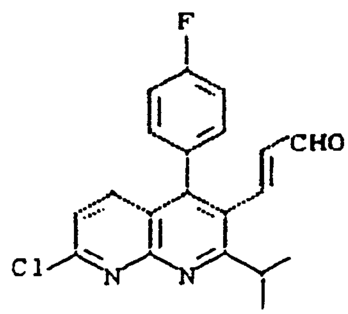

7,8 g (23 mmol) der Verbindung aus Beispiel 5 werden in 20 ml Phosphoroxychlorid auf 75°C erhitzt. Nachdem eine klare Lösung vorliegt wird im Vakuum eingeengt, der Rückstand in Essigester gelöst, auf Eiswasser gegossen und kräftig durchgerührt. Die wäßrige Phase wird noch zweimal mit Essigester extrahiert, die vereinigten organischen Phasen getrocknet und das Lösungsmittel abgezogen. Aus Ether kristallisieren 5,78 g (71 %) farblose Kristalle vom Schmp.: 142°C.

Die in der Tabelle 1 aufgeführten Verbindungen werden nach folgender Vorschrift oder nach Analogieverfahren hergestellt:

Zu einer Suspension von 46 mg (1,55 mmol) 80%igem Natriumhydrid in 5 ml THF tropft man bei 0°C eine Lösung von 167 mg (1,55 mmol) Benzylalkohol in 1 ml THF und rührt 15 Min. nach.

Es wird bei derselben Temperatur eine Lösung von 0,5 g (1,4 mmol) der Verbindung aus Beispiel 19 in 5 ml THF zugetropft und dann 2,5 h bei Raumtemperatur gerührt. Man gibt 1,6 ml Essigsäure in 20 ml Wasser zu, extrahiert 2 mal mit 20 ml Essigester, trocknet die vereinigten organischen Phasen, engt ein und chromatographiert den Rückstand an 20 g Kieselgel 230-400 mesh mit Petrolether/Essigester 6:1→3:1.

Aus Ether/Petrolether kristalliseren 286 mg (49 %) farblose Kristalle vom Schmp.: 182°C.

Tabelle 1

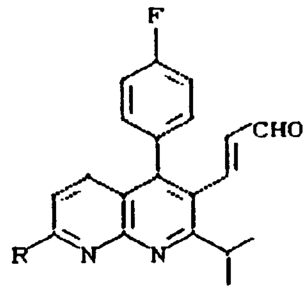

| Beispiel Nr. | R | Reagenz, Base Bedingungen | F (°C) (Lösungsmittel) | Ausbeute (%) |
|---|---|---|---|---|
| 20 | $-OCH_2-C_6H_5$ | $HO-CH_2-C_6H_5$, NaH, 2,5 h, RT, Tetrahydrofuran | 182 Ether/ Petrolether | 49 |
| 21 | $-OCH_3$ | $NaOCH_3$, 2 h, RT, Tetrahydrofuran | 178 $CH_2Cl_2$/ Petrolether | 26 |
| 22 | $-O-CH_2-C(CH_3)_3$ | $HO-CH_2-C(CH_3)_3$, NaH, 10 Min. Rückfluß, Tetrahydrofuran | Öl | 26 |
| 23 | $S-CH_2-C_6H_5$ | $HS-CH_2-C_6H_5$, DBU, 4 h, RT, Tetrahydrofuran | 208 | 61 |
| 24 | $N(CH_3)-CH_2-C_6H_5$ | $H-N(CH_3)-CH_2-C_6H_5$ 1 h, Rückfluß Dioxan | 158 | 31 |

Die in Tabelle 2 aufgeführten Verbindungen wurden in Analogie zur Vorschrift des Beispiels 12 aus den Verbindungen der Beispiele 19, 20, 21, 22, 23 und 24 hergestellt.

Tabelle 2:

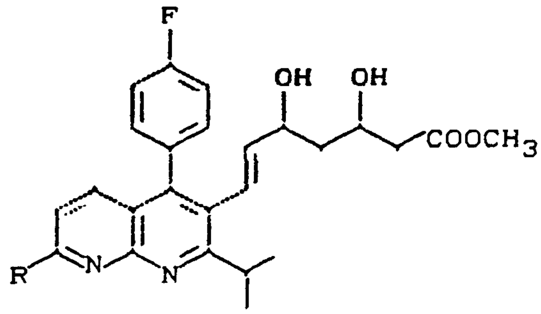

| Beispiel Nr. | R | F (°C) Lösungsmittel | $^1$H-NMR ($CDCl_3$) |
|---|---|---|---|
| 25 | $-O-CH_2-C_6H_5$ | 170 Ether | |
| 26 | $-OCH_3$ | 155 Ether/ Petrolether | |
| 27 | $-O-CH_2-C(CH_3)_3$ | amorph | 1,08 (s, 9H); 2,45 (m, 2H); 3,73 (s, 3H); 4,1 (m, 1H); 4,3 (s, 2H); 4,36 (m, 1H); 6,57 (d, 1H); 7,15 (m, 4H); 7,58 (d, 1H) |
| 28 | $-S-CH_2-C_6H_5$ | 165 | |
| 29 | $-N(CH_3)-CH_2-C_6H_5$ | 134 Ether | |
| 30 | Cl | 207 Ether | |

Die in Tabelle 3 aufgeführten Verbindungen wurden in Analogie zur Vorschrift des Beispiels 7 hergestellt.

Tabelle 3:

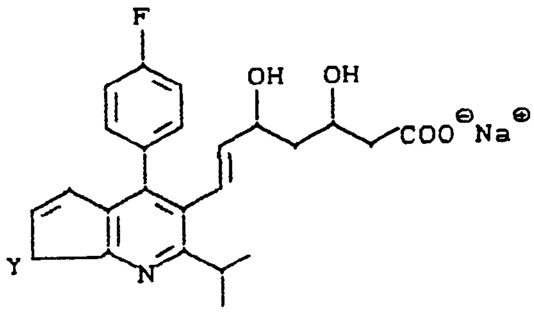

| Bsp.-Nr. | Y | Ausgangs-verbindung | Ausbeute (%) | F °C | FAB-MS |
|---|---|---|---|---|---|
| 31 | 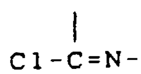 | 30 | 92 | ab 150° Zersetzung | |
| 32 | 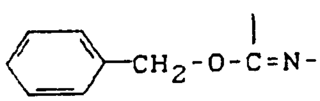 | 25 | 100 | | 575 (M+Na) |
| 33 | 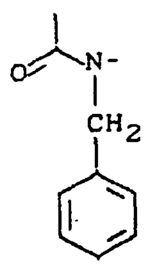 | 10 | 92 | | 554 (M+Na) |

**Patentansprüche**

**Patentansprüche für folgende Vertragsstaaten : AT, BE, CH, DE, DK, FR, GB, GR, IT, LI, LU, NL, SE**

1. Substituierte 1,8-Naphthyridine der allgemeinen Formel

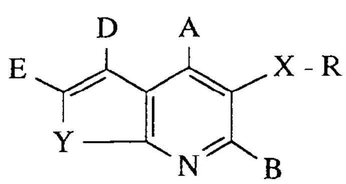

(Ia)

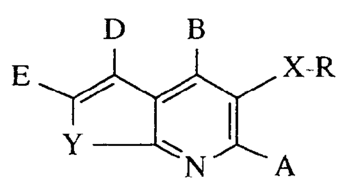

(Ib)

in welcher

A - für Pyridyl oder Pyrimidyl steht, das gegebenenfalls bis zu 2-fach gleich oder verschieden durch Fluor, Chlor, Brom, Trifluormethyl, geradkettiges oder verzweigtes Alkyl mit bis zu 6 Kohlenstoffatomen oder Phenyl Substituiert ist,

- für Phenyl oder Naphthyl steht, das gegebenenfalls bis zu 4-fach gleich oder verschieden substituiert ist durch geradkettiges oder verzweigtes Alkyl, Alkylthio, Alkylsulfonyl, Alkoxy oder Alkoxycarbonyl mit jeweils bis zu 8 Kohlenstoffatomen, welche ihrerseits durch Hydroxy, Alkoxy mit bis zu 4 Kohlenstoffatomen, Phenyl oder durch eine Gruppe der Formel $-NR^1R^2$ substituiert sein können,

worin

$R^1$ und $R^2$ gleich oder verchieden sind und

- Wasserstoff, Phenyl, Phenylsulfonyl, geradkettiges oder verzweigtes Alkyl oder Alkylsulfonyl mit bis zu 6 Kohlenstoffatomen, Benzyl oder Benzylsulfonyl bedeuten, oder

- eine Gruppe der Formel $-COR^3$ bedeuten,

worin

$R^3$ - geradkettiges oder verzweigtes Alkyl oder Alkoxy mit bis zu 6 Kohlenstoffatomen oder Phenyl bedeutet, oder durch Phenyl, Phenyloxy, Fluor, Chlor, Brom, Nitro, Cyano, Trifluormethyl, Trifluormethoxy, Benzyloxy oder durch eine Gruppe der Formel $-NR^1R^2$ substituiert ist,

worin

$R^1$ und $R^2$ die oben angegebene Bedeutung haben,

B - für Cyclopropyl, Cyclobutyl, Cyclopentyl oder Cyclohexyl steht,

- für geradkettiges oder verzweigtes Alkyl mit bis zu 10 Kohlenstoffatomen steht, das gegebenenfalls durch Fluor, Chlor, Brom, Trifluormethyl oder Methylthio substituiert sein kann,

D und E gleich oder verschieden sind und

- für Wasserstoff, Hydroxy, Alkoxy mit bis zu 6 Kohlenstoffatomen, geradkettiges oder verzweigtes Alkyl mit bis zu 8 Kohlenstoffatomen, Phenyl oder für eine Gruppe der Formel $-NR^1R^2$ stehen,

worin

$R^1$ und $R^2$ die oben angegebene Bedeutung haben,

Y - für eine Gruppe der Formel

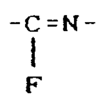 oder 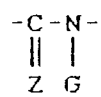

steht

worin

F - Wasserstoff, Hydroxy, Mercapto, Fluor, Chlor oder Brom bedeutet, oder geradkettiges oder verzweigtes Alkyl, Alkoxyoder Alkylthio mit bis zu 8 Kohlenstoffatomen bedeutet, die gegebenenfalls durch Phenyl substituiert sind oder Phenoxy, Benzyloxy oder eine Gruppe der Formel $-NR^1R^2$ bedeutet,

worin

$R^1$ und $R^2$ die oben angegebene Bedeutung haben,

Z - Sauerstoff oder Schwefel bedeutet,

G - Wasserstoff, geradkettiges oder verzweigtes Alkyl oder Alkenyl mit jeweils bis zu 8 Kohlenstoffatomen bedeutet, das gegebenenfalls durch Fluor, Chlor, Brom, Cyano, Alkoxy mit bis zu 6 Kohlenstoffatomen, Phenyl, Phenoxy, Benyzloxy, Pyrryl, Furyl oder durch eine Gruppe der Formel $-NR^1R^2$, $-COR^3$ oder $-COOR^4$ substituiert ist,

worin

$R^1$, $R^2$ und $R^3$ die oben angegebene Bedeutung haben,

$R^4$ - Wasserstoff, geradkettiges oder verzweigtes Alkyl mit bis zu 8 Kohlenstoffatomen bedeutet, das gegebenenfalls durch Hydroxy, Phenyl, Fluor, Chlor oder Brom substituiert ist,

- Phenyl bedeutet, welches seinerseits durch Fluor, Chlor, Brom oder Hydroxy substituiert sein kann,

X - für eine Gruppe der Formel $-CH_2-CH_2-$ oder -CH=CH-steht und

R - für eine Gruppe der Formel

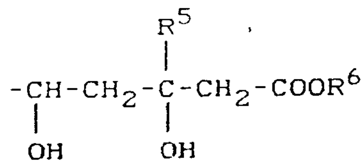 oder 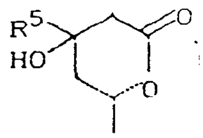

steht,
worin

$R^5$ - Wasserstoff oder geradkettiges oder verzweigtes Alkyl mit bis zu 8 Kohlenstoffatomen bedeutet und

$R^6$ - Wasserstoff oder geradkettiges oder verzweigtes Alkyl mit bis zu 8 Kohlenstoffatomen oder Benzyl bedeutet, oder
- Phenyl oder ein Kation bedeutet

und deren Salze.

2. 1,8-Naphthyridine nach Anspruch 1, wobei

A - für Phenyl steht, das gegebenenfalls bis zu 3-fach gleich oder verschieden substituiert ist durch geradkettiges oder verzweigtes Alkyl mit bis zu 6 Kohlenstoffatomen, welches seinerseits durch Hydroxy, Methoxy, Ethoxy, Propoxy oder Phenyl substituiert sein kann, oder durch Phenyl, Phenoxy, Fluor, Chlor, Brom oder Benzyloxy substituiert ist,

B - für Cyclopropyl, Cyclobutyl, Cyclopentyl oder Cyclohexyl steht,
- für Methyl,Ethyl, Propyl, Isopropyl, Butyl, Isobutyl, tert.Butyl oder Trifluormethyl steht,

D und E gleich oder verschieden sind und
- für Wasserstoff, Hydroxy, Methyl, Ethyl, Propyl, Isopropyl, Methoxy oder Ethoxy stehen,

Y - für eine Gruppe der Formel

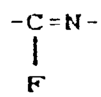 oder 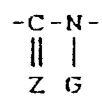

steht,
worin

F - Wasserstoff, Hydroxy, Fluor oder Chlor bedeutet, oder geradkettiges oder verzweigtes Alkyl, Alloxy oder Allylthio mit bis zu 6 Kohlenstoffatomen bedeutet, die gegebenenfalls durch Phenyl substituiert sind, oder Benzyloxy oder eine Gruppe der Formel $-NR^1R^2$ bedeutet, worin

$R^1$ und $R^2$ gleich oder verschieden sind und
- Wasserstoff, geradkettiges oder verzweigtesAlkyl mit bis zu 4 Kohlenstoffatomen oder Benzyl bedeuten,

Z - Sauerstoff oder Schwefel bedeutet,

G - Wasserstoff, geradkettiges oder verzweigtes Alkyl mit bis zu 6 Kohlenstoffatomen bedeutet, das gegebenenfalls durch Fluor, Chlor, Cyano, Alkoxy mit bis zu 4 Kohlenstoffatomen, Phenyl, Benzyloxy oder durch eine Gruppe der Formel $-COR^3$ oder $-COOR^4$ substituiert ist,
worin

$R^3$ - geradkettiges oder verzweigtes Alkyl oder Phenyl bedeutet,

$R^4$ - Wasserstoff, geradkettiges oder verzweigtes Alkyl mit bis zu 6 Kohlenstoffatomen oder Phenyl bedeutet,

X - für eine Gruppe -CH=CH- oder $-CH_2-CH_2$ steht
und

R - für eine Gruppe der Formel

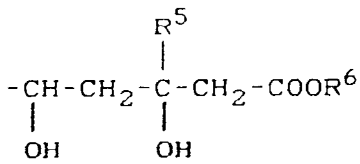

oder

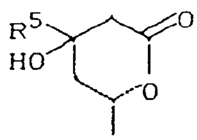

steht,
worin

$R^5$ - Wasserstoff, Methyl, Ethyl, Propyl, Isopropyl, Butyl, Isobutyl oder tert.Butyl bedeutet und

$R^6$ - Wasserstoff, Methyl, Ethyl, Propyl, Isopropyl, Butyl, Isobutyl, tert.Butyl oder Benzyl bedeutet, oder
- ein Natrium-, Kalium-, Calcium-, Magnesiumoder Ammoniumion bedeutet
und deren Salze.

3. Substituierte 1,8-Naphthyridine nach Anspruch 1 zur Bekämpfung von Krankheiten.

4. Verfahren zur Herstellung von 1,8-Naphthyridinen nach Anspruch 1, dadurch gekennzeichnet, daß man Ketone der allgemeinen Formel VIII

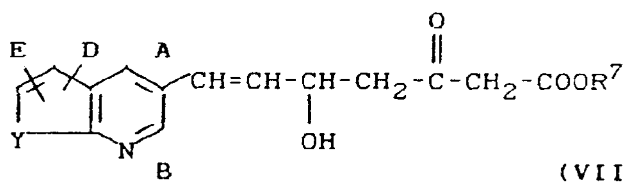

(VIII)

in welcher
A, B, D, E, Y die oben angegebene Bedeutung haben,
und
$R^7$ - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (X = -CH=CH-) nach üblichen Methodenhydriert,
und gegebenenfalls Isomeren trennt.

5. Arzneimittel enthaltend mindestens 1 1,8-Naphthyridin nach Anspruch 1.

6. Arzneimittel nach Anspruch 5 zur Behandlung von Hyperlipoproteinämie, Arteriosklerose oder zur Senkung des Cholesteringehaltes im Blut.

7. Verfahren zur Herstellung eines Arzneimittels nach Anspruch 5, dadurch gekennzeichnet, daß man die 1,8-Naphthyridine gegebenenfalls mit Hilfe üblicher Hilfs- und Trägerstoffe in eine geeignete Applicationsform überführt.

8. Verwendung der 1,8-Naphthyridine nach Anspruch 1 zur Herstellung von Arzneimitteln.

9. Verwendung von 1,8-Naphthyridinen nach Anspruch 1 zur Herstellung von Arzneimitteln zur Behandlung von Hyperlipoproteinämie, Arteriosklerose oder zur Senkung des Cholesteringehaltes im Blut.

**Patentansprüche für folgenden Vertragsstaat : ES**

1. Verfahren zur Herstellung von substituierten 1,8-Naphthyridinen der allgemeinen Formel

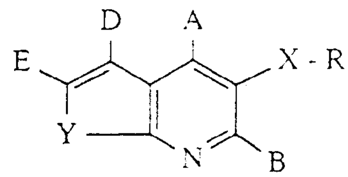

(Ia)

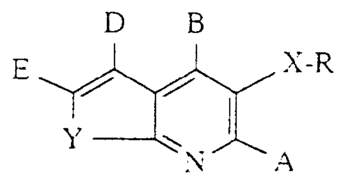

(Ib)

in welcher

A - für Pyridyl oder Pyrimidyl steht, das gegebenenfalls bis zu 2-fach gleichoder verschieden durch Fluor, Chlor, Brom, Trifluormethyl, geradkettiges oder verzweigtes Alkyl mit bis zu 6 Kohlenstoffatomen oder Phenyl substituiert ist,
- für Phenyl oder Naphthyl steht, das gegebenenfallsbis zu 4-fach gleich oder verschieden substituiert ist durch geradkettiges oder verzweigtes Alkyl, Alkylthio, Alkylsulfonyl, Alkoxy oder Alkoxycarbonylmit jeweils bis zu 8 Kohlenstoffatomen, welche ihrerseits durch Hydroxy, Alkoxy mit bis zu 4 Kohlenstoffatomen, Phenyl oder durch eine Gruppe der Formel $-NR^1R^2$ substituiert sein können,
worin
$R^1$ und $R^2$ gleich oder verschieden sind und
- Wasserstoff, Phenyl, Phenyl sullonyl, geradkettiges oder verzweigtes Alkyl oder Alkylsulfonyl mit bis zu 6 Kohlenstoffatomen, Benzyl oder Benzylsulfonyl bedeuten, oder
- eine Gruppe der Formel $-COR^3$ bedeuten,
worin
$R^3$ - geradkettiges oder verzweigtes Alkyl oder Alkoxy mit bis zu 6 Kohlenstoffatomen oder Phenyl bedeutet, oder durch Phenyl, Phenyloxy, Fluor, Chlor, Brom, Nitro, Cyano, Trifluormethyl, Trifluormethoxy, Benzyloxy oder durch eine Gruppe der Formel $-NR^1R^2$ substituiert ist,
worin
$R^1$ und $R^2$ die oben angegebene Bedeutung haben,

B - für Cyclopropyl, Cyclobutyl, Cyclopentyl oder Cyclohexyl steht,
- für geradkettiges oder verzweigtes Alkyl mit bis zu 10 Kohlenstoffatomen steht, das gegebenenfalls durch Fluor, Chlor, Brom, Trifluormethyl oder Methylthio substituiert sein kann,
D und E gleich oder verschieden sind und
- für Wasserstoff, Hydroxy, Alkoxy mit bis zu 6 Kohlenstoffatomen, geradkettiges oder verzweigtes Alkyl mit bis zu 8 Kohlenstoffatomen, Phenyl oder für eine Gruppe der Formel $-NR^1R^2$ stehen,
worin
$R^1$ und $R^2$ die oben angegebene Bedeutung haben,

Y - für eine Gruppe der Formel

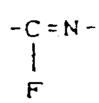

oder

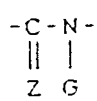

steht
worin

F - Wasserstoff, Hydroxy, Mercapto, Fluor, Chlor oder Brom bedeutet, oder geradkettiges oder verzweigtes Alkyl, Alkoxy oder Alkylthio mit bis zu 8 Kohlenstoffatomen bedeutet, die gegebenenfalls durch Phenyl substituiert sind oder Phenoxy, Benzyloxy oder eine Gruppe der Formel $-NR^1R^2$ bedeutet,
worin

| | |
|---|---|
| | $R^1$ und $R^2$ die oben angegebene Bedeutung haben, |
| Z | - Sauerstoff oder Schwefel bedeutet, |
| G | - Wasserstoff, geradkettiges oder verzweigtes Alkyl oder Alkenyl mit jeweils bis zu 8 Kohlenstoffatomen bedeutet, das gegebenenfalls durch Fluor, Chlor, Brom, Cyano, Alkoxy mit bis zu 6 Kohlenstoffatomen,Phenyl, Phenoxy, Benyzloxy, Pyrryl, Furyl oder durch eine Gruppe der Formel $-NR^1R^2$, $-COR_3$ oder $-COOR^4$ substituiert ist, worin $R^1$, $R^2$ und $R^3$ die oben angegebene Bedeutung haben, |
| $R^4$ | - Wasserstoff, geradkettiges oder verzweigtes Alkyl mit bis zu 8 Kohlenstoffatomen bedeutet, das gegebenenfalls durch Hydroxy, Phenyl, Fluor, Chlor oder Brom substituiert ist, - Phenyl bedeutet , welches seinerseits durch Fluor, Chlor, Brom oder Hydroxy substituiert sein kann, |
| X | - für eine Gruppe der Formel $-CH_2-CH_2-$ oder -CH=-CH-steht und |
| R | - für eine Gruppe der Formel |

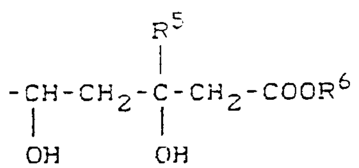

oder

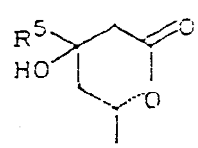

steht,
worin

| | |
|---|---|
| $R^5$ | - Wasserstoff oder geradkettiges oder verzweigtes Alkyl mit bis zu 8 Kohlenstoffatomen bedeutet und |
| $R^6$ | - Wasserstoff oder geradkettiges oder verzweigtes Alkyl mit bis zu 8 Kohlenstoffatomen oder Benzyl bedeutet, oder - Phenyl oder ein Kation bedeutet und deren Salze, dadurch gekennzeichnet, daß man Ketone der allgemeinen Formel VIII |

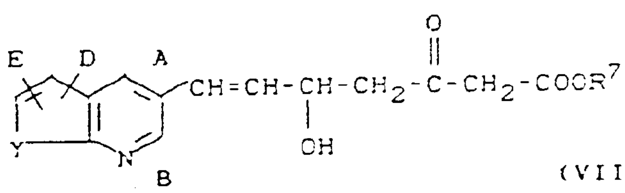

(VIII)

in welcher
A, B, D, E, Y die oben angegebene Bedeutung haben,
und
$R^7$ - für Alkyl steht,
reduziert,
im Fall der Herstellung der Säuren die Esterverseift,
im Fall der Herstellung der Lactone die Carbonsäuren cyclisiert,
in Fall der Herstellung der Salze entweder die Ester oder die Lactone verseift,
im Fall der Herstellung der Ethylenverbindungen ($X = -CH_2-CH_2-$) der Ethenverbindungen (X = -CH=CH-) nach üblichen Methoden hydriert,
und gegebenenfalls Isomeren trennt.

**2.** Verfahren nach Anspruch 1 zur Herstellung von 1,8-Naphthyridinen, wobei

| | |
|---|---|
| A | - für Phenyl steht, das gegebenenfalls bis zu 3-fach gleich oder verschieden substituiert ist durch geradkettiges oder verzweigtes Alkyl mit bis zu 6 Kohlenstoffatomen, welches |

seinerseits durch Hydroxy, Methoxy, Ethoxy, Propoxy oder Phenyl substituiert sein kann, oder durch Phenyl, Phenoxy, Fluor, Chlor, Brom oder Benzyloxy substituiert ist,

B - für Cyclopropyl, Cyclobutyl, Cyclopentyl oder Cyclohexyl steht,
- für Methyl, Ethyl, Propyl, Isopropyl, Butyl, Isobutyl, tert.Butyl oder Trifluormethyl steht,

D und E gleich oder verschieden sind und
- für Wasserstoff, Hydroxy, Methyl, Ethyl, Propyl, Isopropyl, Methoxy oder Ethoxy stehen,

Y - für eine Gruppe der Formel

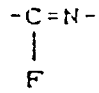

oder

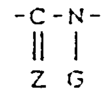

steht,
worin

F - Wasserstoff, Hydroxy, Fluor oder Chlor bedeutet, oder geradkettiges oder verzweigtes Alkyl, Alkoxy oder Alkylthio mit bis zu 6 KohLenstoffatomen bedeutet, die gegebenenfalls durch Phenyl substituiert sind, oder Benzyloxy oder eineGruppe der Formel $-NR^1R^2$ bedeutet, worin
$R^1$ und $R^2$ gleich oder verschieden sind
und
- Wasserstoff, geradkettiges oder verzweigtes Alkyl mit bis zu 4 Kohlenstoffatomen oder Benzyl bedeuten,

Z - Sauerstoff oder Schwefel bedeutet,

G - Wasserstoff, geradkettiges oder verzweigtes Alkyl mit bis zu 6 Kohlenstoffatomen bedeutet, das gegebenenfalls durch Fluor, Chlor, Cyano, Alkoxy mit bis zu 4 Kohlenstoffatomen, Phenyl, Benzyloxy oder durch eine Gruppe der Formel $-COR^3$ oder $-COOR^4$ substituiert ist,
worin

$R^3$ - geradkettiges oder verzweigtes Alkyl oder Phenyl bedeutet,

$R^4$ - Wasserstoff, geradkettiges oder verzweigtes Alkyl mit bis zu 6 Kohlenstoffatomen oder Phenyl bedeutet,

X - für eine Gruppe $-CH=CH-$ oder $-CH_2-CH_2$ steht
und

R - für eine Gruppe der Formel

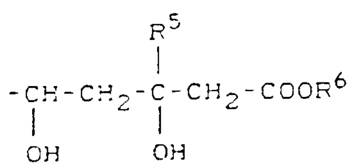

oder

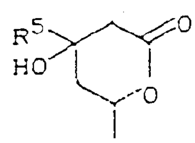

steht,
worin

$R^5$ - Wasserstoff, Methyl, Ethyl, Propyl, Isopropyl, Butyl, Isobutyl oder ter-Butyl bedeutet
und

$R^6$ - Wasserstoff, Methyl, Ethyl, Propyl, Isopropyl, Butyl, Isobutyl, tert.Butyl oder Benzyl bedeutet, oder
- ein Natrium-, Kalium-, Calcium-, Magnesium- oder Ammoniumion bedeutet
und deren Salze.

**3.** Verwendung der 1,8-Naphthyridine nach Anspruch 1 - 2 zur Herstellung von Arzneimitteln.

**4.** Verwendung von 1,8-Naphthyridinen nach Anspruch 1 - 2 zur Herstellung von Arzneimitteln zur Behandlung von Hyperlipoproteinämie, Arteriosklerose oder zur Senkung des Cholesteringehaltes im Blut.

## Claims

**Claims for the following Contrating States : AT, BE, CH, DE, DK, FR, GR, IT, LI, LU, NL, SE**

**1.** Substituted 1,8-naphthyridines of the general formula

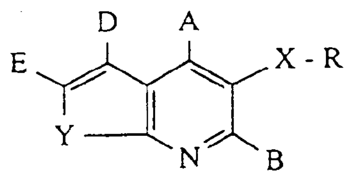

(Ia)

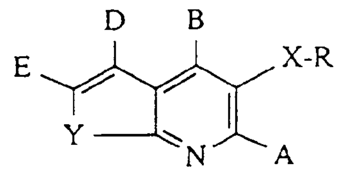

(Ib)

in which

A - represents pyridyl or pyrimidyl, which is optionally monosubstituted or disubstituted by identical or different substituents from the series comprising fluorine, chlorine, bromine, trifluoromethyl, straight-chain or branched alkyl having up to 6 carbon atoms or phenyl,
- represents phenyl or naphthyl, which is optionally monosubstituted to tetrasubstituted by identical or different substituents from the series comprising straight-chain or branched alkyl, alkylthio, alkylsulphonyl, alkoxy or alkoxycarbonyl in each case having up to 8 carbon atoms, which may in turn be substituted by hydroxyl, alkoxy having up to 4 carbon atoms, phenyl or by a group of the formula - $NR^1R^2$,
in which
$R^1$ and $R^2$ are identical or different and
- denote hydrogen, phenyl, phenylsulphonyl, straight-chain or branched alkyl or alkylsulphonyl having up to 6 carbon atoms, benzyl or benzylsulphonyl, or
- denote a group of the formula $-COR^3$,
in which
$R^3$ - denotes straight-chain or branched alkyl or alkoxy having up to 6 carbon atoms or phenyl, or is substituted by phenyl, phenyloxy, fluorine, chlorine, bromine, nitro, cyano, trifluoromethyl, trifluoromethoxy, benzyloxy or by a group of the formula $-NR^1R^2$,
in which
$R^1$ and $R^2$ have the above-mentioned meaning,

B - represents cyclopropyl, cyclobutyl, cyclopentyl or cyclohexyl,
- represents straight-chain or branched alkyl having up to 10 carbon atoms, which may optionally be substituted by fluorine, chlorine, bromine, trifluoromethyl or methylthio,
D and E are identical or different and
- represent hydrogen, hydroxyl, alkoxy having up to 6 carbon atoms, straight-chain or branched alkyl having up to 8 carbon atoms, phenyl or a group of the formula
$-NR^1R^2$,
in which
$R^1$ and $R^2$ have the abovementioned meaning,

Y - represents a group of the formula

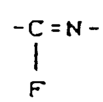

or

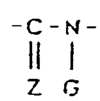

in which

F - denotes hydrogen, hydroxyl, mercapto, fluorine, chlorine or bromine, or denotes straight-chain or branched alkyl, alkoxy or alkylthio having up to 8 carbon atoms, whichare optionally substituted by phenyl, or denotes phenoxy, benzyloxy or a group of the formula
$-NR^1R^2$,
in which

$R^1$ and $R^2$ have the abovementioned meaning,

Z - denotes oxygen or sulphur,

G - denotes hydrogen, straight-chain or branched alkyl or alkenyl in each case having up to 8 carbon atoms, which is optionally substituted by fluorine, chlorine, bromine, cyano, alkoxy having up to 6 carbon atoms, phenyl, phenoxy, benzyloxy, pyrryl, furyl or by a group of the formula $-NR^1R^2$, $-COR^3$ or $-COOR^4$,

in which

$R^1$, $R^2$ and $R^3$ have the abovementioned meaning,

$R^4$ - denotes hydrogen, straight-chain or branched alkyl having up to 8 carbon atoms, which is optionally substituted by hydroxyl, phenyl, fluorine, chlorine or bromine,

- denotes phenyl which may in turn be substituted by fluorine, chlorine, bromine or hydroxyl,

X - represents a group of the formula $-CH_2-CH_2-$ or $-CH=CH-$

and

R - represents a group of the formula

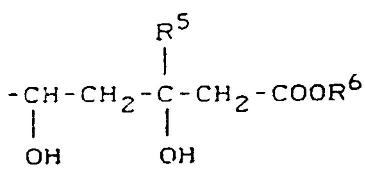

or

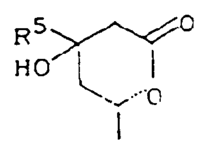

in which

$R^5$ - denotes hydrogen or straight-chain or branched alkyl having up to 8 carbon atoms

and

$R^6$ - denotes hydrogen or straight-chain or branched alkyl having up to 8 carbon atoms, or benzyl, or

- denotes phenyl or a cation

and their salts,

**2.** 1,8-Naphthyridines according to Claim 1, where

A - represents phenyl which is optionally mono-substituted to trisubstituted by identical or different substituents from the series comprising straight-chain or branched alkyl having up to 6 carbon atoms, which may in turn be substituted by hydroxyl, methoxy, ethoxy, propoxy or phenyl,

or is substituted by phenyl, phenoxy, fluorine, chlorine, bromine or benzyloxy,

B - represents cyclopropyl, cyclobutyl, cyclopentyl or cyclohexyl,

- represents methyl, ethyl, propyl, isopropyl, butyl, isobutyl, tert.butyl or trifluoromethyl,

D and E are identical or different and

- represent hydrogen, hydroxyl, methyl, ethyl, propyl, isopropyl, methoxy or ethoxy,

Y - represents a group of the formula

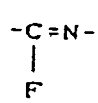

or

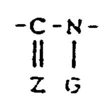

in which

F - denotes hydrogen, hydroxyl, fluorine or chlorine, or denotes straight-chain or branched alkyl, alkoxy or alkylthio having up to 6 carbon atoms, which are optionally substituted by phenyl, or denotes benzyloxy or a group of the formula $-NR^1R^2$, in which

$R^1$ and $R^2$ are identical or different and

- denote hydrogen or straight-chain or branched alkyl having up to 4 carbon atoms, or benzyl,

Z - denotes oxygen or sulphur,

G - denotes hydrogen, straight-chain or branched alkyl having up to 6 carbon atoms, which

is optionally substituted by fluorine, chlorine, cyano, alkoxy having up to 4 carbon atoms, phenyl, benzyloxy or by a group of the formula $-COR^3$ or $-COOR^4$,
in which

| | |
|---|---|
| $R^3$ | - denotes straight-chain or branched alkyl or phenyl, |
| $R^4$ | - denotes hydrogen, straight-chain or branched alkyl having up to 6 carbon atoms or phenyl, |
| X | - represents a group -CH=CH- or $-CH_2-CH_2-$ and |
| R | - represents a group of the formula |

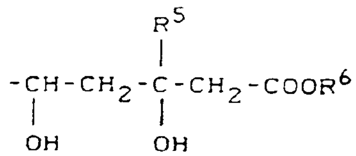

or

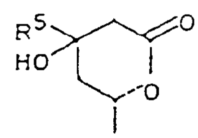

in which

| | |
|---|---|
| $R^5$ | - denotes hydrogen, methyl, ethyl, propyl, isopropyl, butyl, isobutyl or tert.butyl and |
| $R^6$ | - denotes hydrogen, methyl, ethyl, propyl, isopropyl, butyl, isobutyl, tert.butyl or benzyl, or - denotes a sodium, potassium, calcium, magnesium or ammonium ion and their salts. |

**3.** Substituted 1,8-naphthyridines according to Claim 1 for combating diseases.

**4.** Process for the preparation of 1,8-naphthyridines according to Claim 1, characterized in that ketones of the general formula VIII

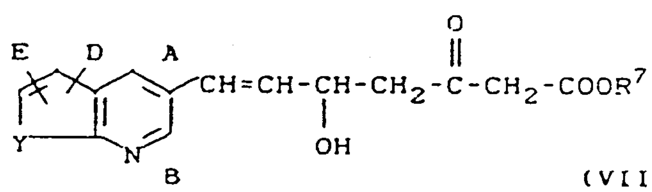

(VIII)

in which
A, B, D, E and Y have the abovementioned meaning,
and
$R^7$ - represents alkyl,
are reduced,
in the case of the preparation of the acids the esters are hydrolyzed,
in the case of the preparation of the lactones the carboxylic acids are cyclized,
in the case of the preparation of the salts either the esters or the lactones are hydrolyzed,
in the case of the preparation of the ethylene compounds ($X = -CH_2-CH_2-$) the ethene compounds (X = -CH=CH-) are hydrogenated according to customary methods,
and, if appropriate, isomers are separated.

**5.** Medicaments containing at least 1 1,8-naphthyridine according to Claim 1.

**6.** Medicaments according to Claim 5 for the treatment of hyperlipoproteinaemia, arteriosclerosis or for lowering the cholesterol content of the blood.

**7.** Process for the production of a medicament according to Claim 5, characterized in that the 1,8-naphthyridines are brought into a suitable form of administration, if appropriate with the aid of customary auxiliaries and excipients.

**8.** Use of the 1,8-naphthyridines according to Claim 1 for the production of medicaments.

9. Use or 1,8-naphthyridines according to Claim 1 for the production of medicaments for the treatment of hyperlipoproteinaemia, arteriosclerosis or for lowering the cholesterol content of the blood.

**Claims for the following Contracting State : ES**

1. Process for the preparation of substituted 1,8- naphthyridines of the general formula

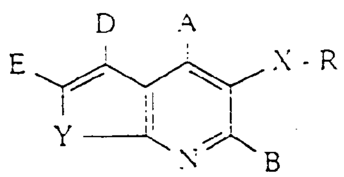

(Ia)

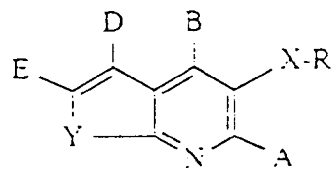

(Ib)

in which

A - represents pyridyl or pyrimidyl, which is optionally monosubstituted or disubstituted by identical or different substituents from the series comprising fluorine, chlorine, bromine, trifluoromethyl, straight-chain or branched alkyl having up to 6 carbon atoms or phenyl,
- represents phenyl or naphthyl, which is optionally monosubstituted to tetrasubstituted by identical or different substituents from the series comprising straight-chain or branched alkyl, alkylthio, alkylsulphonyl, alkoxy or alkoxycarbonyl in each case having up to 8 carbon atoms, which may in turn be substituted by hydroxyl, alkoxy having up to 4 carbon atoms, phenyl or by a group of the formula $-NR^1R^2$,
in which
$R^1$ and $R^2$ are identical or different and
- denote hydrogen, phenyl, phenylsulphonyl, straight-chain or branched alkyl or alkylsulphonyl having up to 6 carbon atoms, benzyl or benzylsulphonyl, or
- denote a group of the formula $-COR^3$,
in which
$R^3$ - denotes straight-chain or branched alkyl or alkoxy having up to 6 carbon atoms or phenyl, or is substituted by phenyl, phenyloxy, fluorine, chlorine, bromine, nitro, cyano, trifluoromethyl, trifluoromethoxy, benzyloxy or by a group of the formula $-NR^1R^2$,
in which
$R^1$ and $R^2$ have the abovementioned meaning,

B - represents cyclopropyl, cyclobutyl, cyclopentyl or cyclohexyl,
- represents straight-chain or branched alkyl having up to 10 carbon atoms, which may optionally be substituted by fluorine, chlorine, bromine, trifluoromethyl or methylthio,
D and E are identical or different and
- represent hydrogen, hydroxyl, alkoxy having up to 6 carbon atoms, straight-chain or branched alkyl having up to 8 carbon atoms, phenyl or a group of the formula $-NR^1R^2$,
in which
$R^1$ and $R^2$ have the abovementioned meaning,

Y - represents a group of the formula

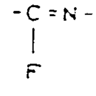

or

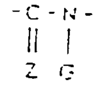

in which

F - denotes hydrogen, hydroxyl, mercapto, fluorine, chlorine or bromine, or denotes straight-chain or branched alkyl, alkoxy or alkylthiohaving up to 8 carbon atoms, which are optionally substituted by phenyl, or denotes phenoxy, benzyloxy or a group of the formula $-NR^1R^2$,

in which
$R^1$ and $R^2$ have the abovementioned meaning,

Z - denotes oxygen or sulphur,

G - denotes hydrogen, straight-chain or branched alkyl or alkenyl in each case having up to 8 carbon atoms, which is optionally substituted by fluorine, chlorine, bromine, cyano, alkoxy having up to 6 carbon atoms, phenyl, phenoxy, benzyloxy, pyrryl, furyl or by a group of the formula $-NR^1R^2$, $-COR^3$ or $-COOR^4$,
in which
$R^1$, $R^2$ and $R^3$ have the abovementioned meaning,

$R^4$ - denotes hydrogen, straight-chain or branched alkyl having up to 8 carbon atoms, which is optionally substituted by hydroxyl, phenyl, fluorine, chlorine or bromine,
- denotes phenyl which may in turn be substituted by fluorine, chlorine, bromine or hydroxyl,

X - represents a group of the formula $-CH_2-CH_2-$ or $-CH=CH-$
and

R - represents a group of the formula

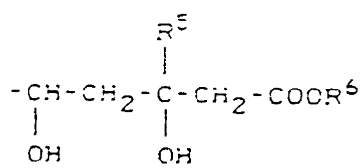
or
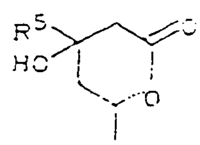

in which

$R^5$ - denotes hydrogen or straight-chain or branched alkyl having up to 8 carbon atoms
and

$R^6$ - denotes hydrogen or straight-chain or branched alkyl having up to 8 carbon atoms, or benzyl, or
- denotes phenyl or a cation

and their salts,
characterized in that ketones of the general formula VIII

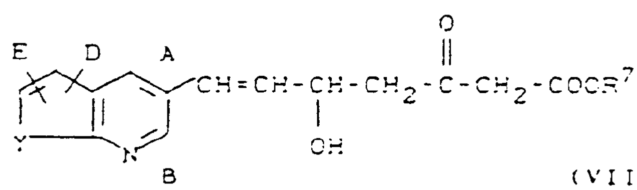
(VIII)

in which
A, B, D, E and Y have the abovementioned meaning,
and
$R^7$ - represents alkyl,
are reduced,
in the case of the preparation of the acids the esters are hydrolyzed,
in the case of the preparation of the lactones the carboxylic acids are cyclized,
in the case of the preparation of the salts either the esters or the lactones are hydrolyzed,
in the case of the preparation of the ethylene compounds ($X = -CH_2-CH_2-$) the ethene compounds ($X = -CH=CH-$) are hydrogenated according to customary methods,
and, if appropriate, isomers are separated.

**2.** Process according to Claim 1 for the preparation of 1,8-Naphthyridines where

A - represents phenyl which is optionally mono-substituted to trisubstituted by identical or

different substituents from the series comprising straight-chain or branched alkyl having up to 6 carbon atoms, which may in turn be substituted by hydroxyl, methoxy, ethoxy, propoxy or phenyl,
or is substituted by phenyl, phenoxy, fluorine, chlorine, bromine or benzyloxy,

B - represents cyclopropyl, cyclobutyl, cyclopentyl or cyclohexyl,
- represents methyl, ethyl, propyl, isopropyl, butyl, isobutyl, tert.butyl or trifluoromethyl,

D and E are identical or different and
- represent hydrogen, hydroxyl, methyl, ethyl, propyl, isopropyl, methoxy or ethoxy,

Y - represents a group of the formula

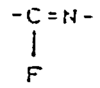 or 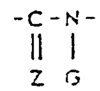

in which

F - denotes hydrogen, hydroxyl, fluorine or chlorine, or denotes straight-chain or branched alkyl, alkoxy or alkylthio having up to 6 carbon atoms, which are optionally substituted by phenyl, or denotes benzyloxy or a group of the formula $-NR^1R^2$, in which

$R^1$ and $R^2$ are identical or different and - denote hydrogen or straight-chain or branched alkyl having up to 4 carbon atoms, or benzyl,

Z - denotes oxygen or sulphur,

G - denotes hydrogen, straight-chain or branched alkyl having up to 6 carbon atoms, which is optionally substituted by fluorine, chlorine, cyano, alkoxy having up to 4 carbon atoms, phenyl, benzyloxy or by a group of the formula $-COR^3$ or $-COOR^4$,
in which

$R^3$ - denotes straight-chain or branched alkyl or phenyl,

$R^4$ - denotes hydrogen, straight-chain or branched alkyl having up to 6 carbon atoms or phenyl,

X - represents a group $-CH=CH-$ or $-CH_2-CH_2-$
and

R - represents a group of the formula

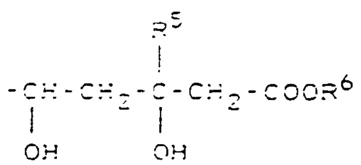 or 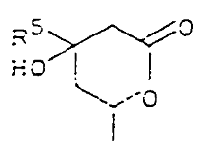

in which

$R^5$ - denotes hydrogen, methyl, ethyl, propyl, isopropyl, butyl, isobutyl or tert.butyl
and

$R^6$ - denotes hydrogen, methyl, ethyl, propyl, isopropyl, butyl, isobutyl, tert.butyl or benzyl,
or
- denotes a sodium, potassium, calcium, magnesium or ammonium ion

**3.** Use of the 1,8-naphthyridines according to Claim 1-2 for the production of medicaments.

**4.** Use of 1,8-naphthyridines according to Claim 1-2 for rhe production of medicaments for the treatment of hyperlipoproteinaemia, arteriosclerosis or for lowering the cholesterol content of the blood.

## Revendications

**Revendications pour les Etats contractants suivants : AT, BE, CH, DE, DK, FR, GB, GR, IT, LI, LU, NL, SE**

**1.** 1,8-naphtyridines subustituées de formule générale

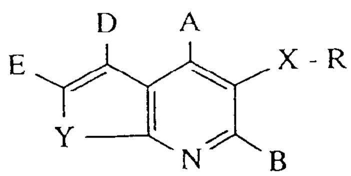

(Ia)

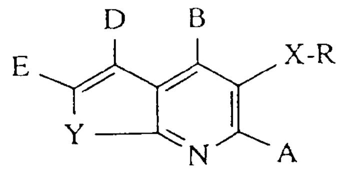

(Ib)

dans laquelle

A - représente un groupe pyridyle ou pyrimidyle qui porte le cas échéant jusqu'à 2 substituants, identiques ou différents, fluoro, chloro, bromo, trifluorométhyle, alkyle à chaîne droite ou ramifiée ayant jusqu'à 6 atomes de carbone, ou phényle,
- un groupe phényle ou naphtyle qui porte le cas échéant jusqu'à 4 substituants, identiques ou différents, alkyle à chaîne droite ou ramifiée, alkylthio, alkylsulfonyle, alkoxy ou alkoxycarbonyle ayant chacun jusqu'à 8 atomes de carbone, pouvant être substitués quant à eux par un groupe hydroxy, alkoxy ayant jusqu'à 4 atomes de carbone, phényle, ou par un groupe de formule $-NR^1R^2$,
dans laquelle
$R^1$ et $R^2$ sont identiques ou différents et représentent
- l'hydrogène, un groupe phényle, phénylsulfonyle, alkyle à chaîne droite ou ramifiée ou alkylsulfonyle ayant jusqu'à 6 atomes de carbone, benzyle ou benzylsulfonyle, ou bien
-un groupe de formule $-COR^3$ dans laquelle
$R^3$ - est un groupe alkyle ou alkoxy à chaîne droite ou ramifiée ayant jusqu'à 6 atomes de carbone ou un groupe phényle, ou est substitué par un groupe phényle, phényloxy, du fluor, du chlore, du brome, un groupe nitro, cyano, trifluorométhyle, trifluorométhoxy, benzyloxy, ou par un groupe de formule $-NR^1R^2$
dans laquelle
$R^1$ et $R^2$ ont la définition indiquée ci-dessus,

B - est un groupe cyclopropyle, cyclobutyle, cyclopentyle ou cyclohexyle,
- un groupe alkyle à chaîne droite ou ramifiée ayant jusqu'à 10 atomes de carbone, qui peut être substitué le cas échéant par du fluor, du chlore, du brome, un radical trifluorométhyle ou méthylthio,

D et E sont identiques ou différents et représentent
- l'hydrogène un groupe hydroxy, alkoxy ayant jusqu'à 6 atomes de carbone, alkyle à chaîne droite ou ramifiée ayant jusqu'à 8 atomes de carbone, phényle, ou un groupe de formule $-NR^1R^2$,
dans laquelle
$R^1$ et $R^2$ ont la définition indiquée ci-dessus,

Y - est un groupe de formule

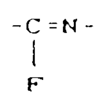

ou

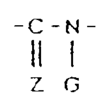

dans laquelle

F - représente l'hydrogène, un groupe hydroxy, mercapto, le fluor, le chlore ou le brome, ou un groupe alkyle, alkoxy ou alkylthio à chaîne droite ou ramifiée ayant jusqu'à 8 atomes de carbone, qui sont éventuellement substitués par un radical phényle, ou un groupe phénoxy, benzyloxy ou un groupe de formule $-NR^1R^2$, dans laquelle $R^1$ et $R^2$ ont la définition indiquée ci-dessus,

Z - représente l'oxygène ou le soufre,

G - est l'hydrogène, un groupe alkyle ou alcényle à chaîne droite ou ramifiée ayant chacun jusqu'à 8 atomes de carbone, qui est substitué le cas échéant par du fluor, du chlore, du brome, un radical cyano, alkoxy ayant jusqu'à 6 atomes de carbone, phényle, phénoxy, benzyloxy, pyrryle, furyle, ou par un groupe de formule $-NR^2R^2$, $-COR^3$ ou $-COOR^4$, où $R^1$, $R^2$ et $R^3$ ont la définition indiquée ci-dessus,

$R^4$ - représente l'hydrogène, un groupe alkyle à chaîne droite ou ramifiée ayant jusqu'à 8 atomes de carbone, qui est substitué le cas échéant par un radical hydroxy, phényle, du fluor, du chlore ou du brome,
- un groupe phényle qui peut être substitué quant à lui par du fluor, du chlore, du brome ou un radical hydroxy,

X - est un groupe de formule $-CH_2-CH_2-$ ou $-CH=CH-$ et

R - est un groupe de formule

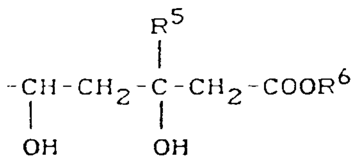

ou

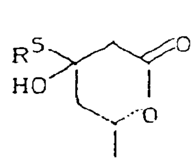

où

$R^5$ - représente l'hydrogène ou un groupe alkyle à chaîne droite ou ramifiée ayant jusqu'à 8 atomes de carbone et

$R^6$ - est l'hydrogène ou un groupe alkyle à chaîne droite ou ramifiée ayant jusqu'à 8 atomes de carbone ou un groupe benzyle, ou bien
- un groupe phényle ou un cation,

et leurs sels.

**2.** 1,8-naphtyridines suivant la revendication 1, dans lesquelles

A - est un groupe phényle qui porte le cas échéant jusqu'à 3 substituants identiques ou différents qui sont des groupes alkyle à chaîne droite ou ramifiée ayant jusqu'à 6 atomes de carbone, qui peuvent être substitués quant à eux par un radical hydroxy, méthoxy, éthoxy, propoxy ou phényle,
ou par un groupe phényle, phénoxy, du fluor, du chlore, du brome ou un groupe benzyloxy,

B - est un groupe cyclopropyle, cyclobutyle, cyclopentyle ou cyclohexyle,
-un groupe méthyle, éthyle, propyle, isopropyle, butyle, isobutyle, tertio-butyle ou trifluorométhyle,

D et E sont identiques ou différents et représentent
- l'hydrogène, un groupe hydroxy, méthyle, éthyle, propyle, isopropyle, méthoxy ou éthoxy,

Y - est un groupe de formule

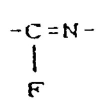

ou

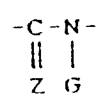

dans laquelle

F - est l'hydrogène, un groupe hydroxy, du fluor ou du chlore, ou un groupe alkyle, alkoxy ou alkylthio à chaîne droite ou ramifiée avec jusqu'à 6 atomes de carbone, qui sont substitués le cas échéant par un radical phényle, ou bien un

groupe benzyloxy ou un groupe de formule $-NR^1R^2$, dans laquelle
$R^1$ et $R^2$ sont identiques ou différents et représentent
- de l'hydrogène, un groupe alkyle à chaîne droite ou ramifiée ayant jusqu'à 4 atomes de carbone ou un groupe benzyle,

Z - est l'oxygène ou le soufre,

G - représente l'hydrogène, un groupe alkyle à chaîne droite ou ramifiée ayant jusqu'à 6 atomes de carbone, qui est substitué le cas échéant par du fluor, du chlore, un radical cyano, alkoxy ayant jusqu'à 4 atomes de carbone, phényle, benzyloxy ou par un groupe de formule $-COR^3$ ou $-COOR^4$,
dans laquelle

$R^3$ - désigne un groupe alkyle à chaîne droite ou ramifiée ou phényle,

$R^4$ - représente l'hydrogène, un groupe alkyle à chaîne droite ou ramifiée ayant jusqu'à 6 atomes de carbone ou un groupe phényle,

X - est un groupe -CH=CH- ou $-CH_2-CH_2$
et

R - est un groupe de formule

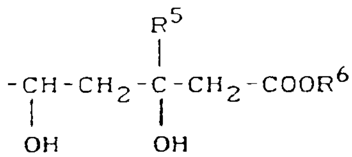

ou

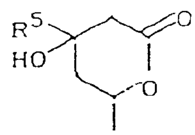

dans laquelle

$R^5$ - représente l'hydrogène, un groupe méthyle, éthyle, propyle, isopropyle, butyle, isobutyle ou tertio-butyle,
et

$R^6$ - représente l'hydrogène, un groupe méthyle, éthyle, propyle, isopropyle, butyle, isobutyle, tertio-butyle ou benzyle, ou bien
- un ion sodium, potassium, calcium, magnésium ou ammonium,
et leur sels.

3. 1,8-naphtyridines substituées suivant la revendication 1, destinées à combattre des maladies.

4. Procédé de production de 1,8-naphtyridines suivant la revendication 1, caractérisé en ce qu'on réduit des cétones de formule générale (VIII)

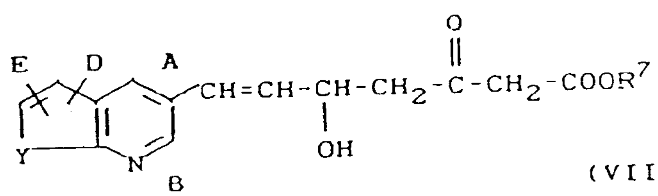

(VIII)

dans laquelle
A, B, D, E, Y ont la définition indiquée ci-dessus
et
$R^7$ - est un groupe alkyle,
dans le cas de la production des acides, on saponifie les esters,
dans le cas de la production des lactones, on cyclise les acides carboxyliques,
dans le cas de la production des sels, on saponifie ou bien les esters ou bien les lactones,
dans le cas de la production des composés éthyléniques ($X = -CH_2-CH_2-$), on hydrogène les composés d'éthène (X = -CH=CH-) par des procédés classiques,
et le cas échéant on sépare les isomères.

5. Médicament contenant au moins une 1,8-naphtyridine suivant la revendication 1.

6. Médicament suivant la revendication 5, destiné au traitement de l'hyperlipoprotéinémie, de l'artériosclérose, ou à abaisser le taux de cholestérol dans le sang.

7. Procédé de préparation d'un médicament suivant la revendication 5, caractérisé en ce qu'on transforme les 1,8-naphtyridines, le cas échéant à l'aide de substances auxiliaires et de supports classiques, en une forme d'application appropriée.

8. Utilisation des 1,8-naphtyridines suivant la revendication 1 pour la préparation de médicaments.

9. Utilisation de 1,8-naphtyridines suivant la revendication 1 pour la préparation de médicaments destinés au traitement de l'hyperlipoprotéinémie, de l'artériosclérose, ou à l'abaissement du taux de cholestérol dans le sang.

**Revendications pour l'Etat contractant suivant : ES**

1. Procédé de production de 1,8-naphtyridines substituées de formule générale

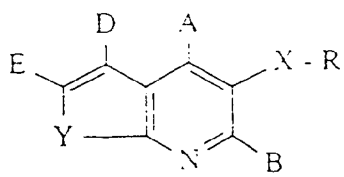

(Ia)

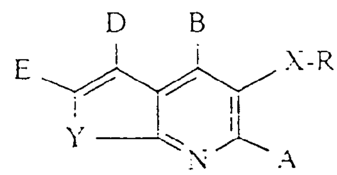

(Ib)

dans laquelle

A - représente un groupe pyridyle ou pyrimidyle qui porte le cas échéant jusqu'à deux substituants, identiques ou différents, fluoro, chloro, bromo, trifluorométhyle, alkyle à chaîne droite ou ramifiée ayant jusqu'à 6 atomes de carbone, ou phényle,
- un groupe phényle ou naphtyle qui porte le cas échéant jusqu'à 4 substituants, identiques ou différents, alkyle à chaîne droite ou ramifiée, alkylthio, alkylsulfonyle, alkoxy ou alkoxycarbonyle ayant chacun jusqu'à 8 atomes de carbone, pouvant être substitués quant à eux par un groupe hydroxy, alkoxy ayant jusqu'à 4 atomes de carbone, phényle, ou par un groupe de formule $-NR^1R^2$, dans laquelle
$R^1$ et $R^2$ sont identiques ou différents et représentent
- l'hydrogène, un groupe phényle, phénylsulfonyle, alkyle à chaîne droite ou ramifiée ou alkylsulfonyle ayant jusqu'à 6 atomes de carbone, benzyle ou benzylsulfonyle, ou bien
- un groupe de formule $-COR^3$
dans laquelle
$R^3$ - est un groupe alkyle ou alkoxy à chaîne droite ou ramifiée ayant jusqu'à 6 atomes de carbone ou un groupe phényle, ou est substitué par un groupe phényle, phényloxy, du fluor, du chlore, du brome, un groupe nitro, cyano, trifluorométhyle, trifluorométhoxy, benzyloxy, ou par un groupe de formule $-NR^1R^2$ dans laquelle
$R^1$ et $R^2$ ont la définition indiquée ci-dessus,

B - est un groupe cyclopropyle, cyclobutyle, cyclopentyle ou cyclohexyle,
- un groupe alkyle à chaîne droite ou ramifiée ayant jusqu'à 10 atomes de carbone, qui peut être substitué le cas échéant par du fluor, du chlore, du brome, un radical trifluorométhyle, ou méthylthio,

D et E sont identiques ou différents et représentent
- l'hydrogène, un groupe hydroxy, alkoxy ayant jusqu'à 6 atomes de carbone, alkyle à chaîne droite ou ramifiée ayant jusqu'à 8 atomes de carbone, phényle, ou un groupe de formule $-NR^1R^2$,
dans laquelle
$R^1$ et $R^2$ ont la définition indiquée ci-dessus,

Y - est un groupe de formule

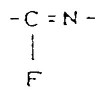 ou 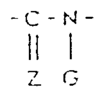

dans laquelle

F - représente l'hydrogène, un groupe hydroxy, mercapto, le fluor, le chlore ou le brome, ou un groupe alkyle, alkoxy ou alkylthio à chaîne droite ou ramifiée ayant jusqu'à 8 atomes de carbone, qui sont éventuellement substitués par un radical phényle, ou un groupe phénoxy, benzyloxy ou un groupe de formule $-NR^1R^2$,
dans laquelle
$R^1$ et $R^2$ ont la définition indiquée ci-dessus,

Z - représente l'oxygène ou le soufre,

G - est l'hydrogène, un groupe alkyle ou alcényle à chaîne droite ou ramifiée ayant chacun jusqu'à 8 atomes de carbone, qui est substitué le cas échéant par du fluor, du chlore, du brome, un radical cyano, alkoxy ayant jusqu'à 6 atomes de carbone, phényle, phénoxy, benzyloxy, pyrryle, furyle, ou par un groupe de formule $-NR^2R^2$, $-COR^3$ ou $-COOR^4$, où $R^1$, $R^2$ et $R^3$ ont la définition indiquée ci-dessus,

$R^4$ - représente l'hydrogène, un groupe alkyle à chaîne droite ou ramifiée ayant jusqu'à 8 atomes de carbone, qui est substitué le cas échéant par un radical hydroxy, phényle, du fluor, du chlore ou du brome,
- un groupe phényle qui peut être substitué quant à lui par du fluor, du chlore, du brome ou un radical hydroxy,

X - est un groupe de formule $-CH_2-CH_2-$ ou $-CH=CH-$
et

R - est un groupe de formule

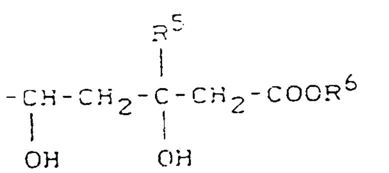 ou 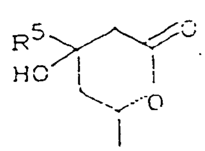 ,

où

$R^5$ - représente l'hydrogène ou un groupe alkyle à chaîne droite ou ramifiée ayant jusqu'à 8 atomes de carbone
et

$R^6$ - est l'hydrogène ou un groupe alkyle à chaîne droite ou ramifiée ayant jusqu'à 8 atomes de carbone ou un groupe benzyle, ou bien
- un groupe phényle ou un cation,
et de leurs sels

caractérisé en ce qu'on réduit des cétones de formule générale (VIII)

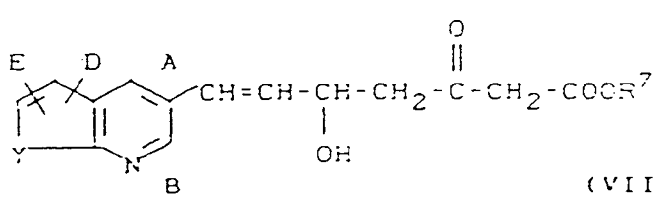

(VIII)

dans laquelle
A, B, D, E, Y ont la définition indiquée ci-dessus,
et
$R^7$ - est un groupe alkyle,

dans le cas de la production d'acide, on saponifie l'ester,

dans le cas de la production des lactones, on cyclise l'acide carboxylique,

dans le cas de la production de sels, on saponifie ou bien les esters ou bien les lactones,

dans le cas de la production des composés éthyléniques ($X = -CH_2-CH_2-$), on hydrogène les composés d'éthène ($X = -CH=CH-$) par des procédés classiques, et on sépare éventuellement les isomères.

**2.** Procédé suivant la revendication 1, pour la production de 1,8-naphtyridines dans lesquelles

A - est un groupe phényle qui porte le cas échéant jusqu'à 3 substituants identiques ou différents qui sont des groupes alkyle à chaîne droite ou ramifiée ayant jusqu'à 6 atomes de carbone, qui peuvent être substitués quant à eux par un radical hydroxy, méthoxy, éthoxy, propoxy ou phényle, ou par un groupe phényle, phénoxy, du fluor, du chlore, du brome ou un groupe benzyloxy,

B - est un groupe cyclopropyle, cyclobutyle, cyclopentyle ou cyclohexyle,
- un groupe méthyle, éthyle, propyle, isopropyle, butyle, isobutyle, tertio-butyle ou trifluorométhyle,

D et E sont identiques ou différents et représentent
- l'hydrogène, un groupe hydroxy, méthyle, éthyle, propyle, isopropyle, méthoxy ou éthoxy,

Y - est un groupe de formule

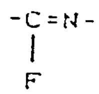

ou

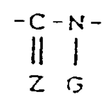

dans laquelle

F - est l'hydrogène, un groupe hydroxy, du fluor ou du chlore, ou un groupe alkyle, alkoxy ou alkylthio à chaîne droite ou ramifiée avec jusqu'à 6 atomes de carbone, qui sont substitués le cas échéant par un radical phényle, ou bien un groupe benzyloxy ou un groupe de formule $-NR^1R^2$, dans laquelle
$R^1$ et $R^2$ sont identiques ou différents et représentent
- de l'hydrogène, un groupe alkyle à chaîne droite ou ramifiée ayant jusqu'à 4 atomes de carbone ou un groupe benzyle,

Z - est l'oxygène ou le soufre,

G - représente l'hydrogène, un groupe alkyle à chaîne droite ou ramifiée ayant jusqu'à 6 atomes de carbone, qui est substitué le cas échéant par du fluor, du chlore, un radical cyano, alkoxy ayant jusqu'à 4 atomes de carbone, phényle, benzyloxy ou par un groupe de formule $-COR^3$ ou $-COOR^4$, dans laquelle

$R^3$ - désigne un groupe alkyle à chaîne droite ou ramifiée ou phényle,

$R^4$ - représente l'hydrogène, un groupe alkyle à chaîne droite ou ramifiée ayant jusqu'à 6 atomes de carbone ou un groupe phényle,

X - est un groupe $-CH=CH-$ ou $-CH_2-CH_2$
et

R - est un groupe de formule

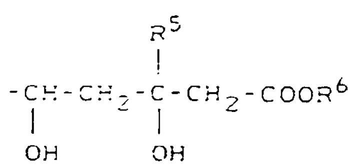

ou

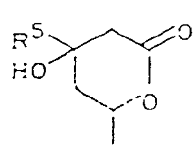

dans laquelle

$R^5$ - représente l'hydrogène, un groupe méthyle, éthyle, propyle, isopropyle, butyle, isobutyle ou tertio-butyle,
et

$R^6$ - représente l'hydrogène, un groupe méthyle, éthyle, propyle, isopropyle, butyle, isobutyle, tertio-butyle ou benzyle, ou bien
- un ion sodium, potassium, calcium, magnésium ou ammonium,

et de leur sels.

3. Utilisation des 1,8-naphtyridines suivant les revendications 1 et 2 pour la préparation de médicaments.

4. Utilisation de 1,8-naphtyridines suivant les revendications 1 et 2 pour la préparation de médicaments destinés au traitement de l'hyperlipoprotéinémie, de l'artériosclérose ou à l'abaissement du taux de cholestérol dans le sang.